(19) Europäisches Patentamt
European Patent Office
Office européen des brevets

(11) EP 3 747 930 B1

(12) **EUROPEAN PATENT SPECIFICATION**

(45) Date of publication and mention of the grant of the patent:
**15.05.2024 Bulletin 2024/20**

(21) Application number: **19747178.2**

(22) Date of filing: **31.01.2019**

(51) International Patent Classification (IPC):
***C08G 65/336*** *(2006.01)* ***C07C 41/18*** *(2006.01)*
***C07F 7/18*** *(2006.01)* ***C08G 65/323*** *(2006.01)*
***C08L 71/00*** *(2006.01)* ***C09D 5/16*** *(2006.01)*
***C09D 171/00*** *(2006.01)* ***C08G 65/22*** *(2006.01)*

(52) Cooperative Patent Classification (CPC):
**C08G 65/007; C07F 7/1804; C07F 7/1876; C08G 65/226; C08G 65/336; C09D 5/1675; C09D 171/00**

(86) International application number:
**PCT/JP2019/003488**

(87) International publication number:
**WO 2019/151442 (08.08.2019 Gazette 2019/32)**

(54) **FLUORO (POLY)ETHER GROUP-CONTAINING SILANE COMPOUND**

FLUOR(POLY)ETHERGRUPPENHALTIGE SILANVERBINDUNG

COMPOSÉ SILANE COMPRENANT UN GROUPE FLUORO(POLY)ÉTHER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.02.2018 JP 2018017571**

(43) Date of publication of application:
**09.12.2020 Bulletin 2020/50**

(73) Proprietor: **DAIKIN INDUSTRIES, LTD.**
**Osaka-Shi, Osaka 530-0001 (JP)**

(72) Inventors:
- **MITSUHASHI, Hisashi**
  **Osaka-shi, Osaka 530-8323 (JP)**
- **NOMURA, Takashi**
  **Osaka-shi, Osaka 530-8323 (JP)**
- **NAITOU, Masato**
  **Osaka-shi, Osaka 530-8323 (JP)**
- **TAKANO, Shinya**
  **Osaka-shi, Osaka 530-8323 (JP)**
- **WATANABE, Yuusuke**
  **Osaka-shi, Osaka 530-8323 (JP)**
- **OZAWA, Kaori**
  **Osaka-shi, Osaka 530-8323 (JP)**
- **HUPFIELD, Peter**
  **40211 Dusseldorf (DE)**

(74) Representative: **Hoffmann Eitle**
**Patent- und Rechtsanwälte PartmbB**
**Arabellastraße 30**
**81925 München (DE)**

(56) References cited:

**EP-A1- 1 923 434**
**EP-A1- 3 085 749**
**EP-A1- 3 345 912**
**EP-A1- 3 578 586**
**EP-A1- 3 747 931**
**WO-A1-2015/200003**
**WO-A1-2017/038305**
**WO-A1-2018/143433**
**JP-A- 2002 348 370**
**JP-A- 2006 113 134**
**JP-A- 2013 244 470**
**JP-A- 2014 214 194**
**JP-A- 2015 168 785**
**JP-A- 2016 027 156**
**JP-A- 2016 037 541**
**JP-A- 2016 094 567**
**JP-A- 2017 048 370**
**JP-A- 2017 125 193**

- **ITAMI Y ET AL: "The influence of perfluoropolyether silane structural modification on performance when used as surface treatment agents", SURFACE COATINGS INTERNATIONAL. PART B: COATINGS TRANSACTIONS, SPRINGER, BOSTON, US, vol. 89, no. B3, 1 September 2006 (2006-09-01), pages 255-258, XP009125609, ISSN: 1476-4865, DOI: 10.1007/BF02699669**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 747 930 B1

## Description

Technical Field

The present disclosure relates to a fluoro(poly)ether group-containing silane compound.

Background Art

Certain fluorine-containing silane compounds are known to be able to provide excellent water-repellency, oil-repellency or antifouling property when used for a surface treatment of a base material. For example, JP-A-2015-221888 discloses a surface treatment composition containing a perfluoro(poly)ether group-containing silane compound having a perfluoro(poly)ether group in the molecular backbone and having a hydrolyzable group bonded to a Si atom at its molecular end or end portion.

WO 2015/200003 discloses silicon-containing polymers for protective coatings, which polymers are of a general formula $R_f{}^1R_f{}^2R_h$-$[Q]_x$-[Z] -I, wherein $R_f{}^1R_f{}^2R_h$- includes a perfluoroalkyl polyether (PFPE) moiety, and Q is polyethylene backbone moiety having -$SiX_4$ groups in the side chains, wherein at least one X is a hydrolyzable group.

WO 2017/038305 relates to perfluoro(poly)ether group-containing silane compounds of the formula (Rf-PFPE)$_\beta$-X-$(CR^a{}_kR^b{}_lR^c{}_m)_\alpha$ or $(R^a{}_kR^a{}_lR^a{}_mC)_\alpha$-X-PFPE-X-$(CR^a{}_k\ R^b{}_lR^c{}_m)_\alpha$ and the use thereof as a surface treatment agent to provide water and oil repellency and antifouling properties to a substrate.

EP-A-1 923 434 describes a coating composition comprising (A) at least one fluorine-containing organosilicon compound of the formula $Rf^1$-$QZ^1A_\alpha$ (1) or $A_\alpha Z^1Q$-$Rf^2$-(Q-$Z^2$-Q-$Rf^2)_x$-$QZ^1A_\alpha$ (2), and (B) a fluorine-containing carboxylic acid having a Mw of 100-10,000 and a fluorine content of ≥ 25 wt%, which composition can be cured rapidly even at room temperature to produce water-repellent and antifouling coatings.

JP-A-2002-348370 defines a perfluoropolyether-modified silane of the formula $F(C_xF_{2x}O)_mC_yF_{2y}$-Q-Z wherein Q is a divalent organic group and Z is a monovalent group of specified formula (2), which polymer can form cured films having water- and oil-repellency, mold-releasing properties and antifouling properties.

EP-A-3 085 749 addresses a fluoropolyether-containing polymer-modified silane of a general formula (1) including a monovalent fluorooxyalkyl- or divalent fluorooxyalkylenecontaining polymer residue, and a plurality of silane containing groups, which polymer forms a film having water and oil repellency and abrasion resistance.

EP-A-3 747 931 relates to an electronic device comprising, on the surface thereof, a surface-treating layer formed of a (poly)ether group-containing silane compound of any of the formulae (A1), (A2), (B1), (B2), (C1), or (C2) including a group $-(OC_6F_{12})_a-(OC_5F_{10})_b-(OC_4F_8)_c-(OC_3X^F{}_6)_d-(OC_2F_4)_e-(OCF_2)_f-$ having at least one branched structure.

Y. Itami et al., Surface Coatings Int. Part B : Coatings, 89, 255-258 (2006) reports on the influence of perfluoropolyether (PFPE) silane structural modification on performance when used as surface treatment agents.

EP-A-3 345 912 discloses a fluorinated ether compound of the formula $A^1$-O-$(R^{f1}O)_{m1}$-$Q^1$-$[C(O)N(R^1)]_{p1}$-$R^{11}$-C[-$R^{12}$-$SiR^{13}{}_{n1}X^1{}_{3-n1}]_3$ (1) which preferably have a poly(oxyperfluoroalkylene) chain with an ether bond present at middle in a perfluoroalkyl chain, and which exhibit high lubricity, water/oil repellency and stain removability.

Summary of Invention

Technical Problem

A layer formed of the surface treatment composition (surface-treating agent) as described above (sometimes referred to as a surface-treating layer) is sometimes required to have durability against ultraviolet rays (UV) (sometimes referred to as "ultraviolet durability" or "UV durability").

The present disclosure relates to a silane compound containing a fluoropolyether group (hereinafter, sometimes referred to as "the present silane compound") that can contribute to the formation of a surface-treating layer having good UV durability.

Solution to Problem

The present invention provides a compound which is a fluoro(poly)ether group-containing silane compound of any of the formulae (A1), (A2), (B1), (B2), (C1) or (C2):

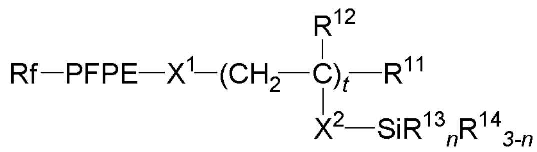
(A1)

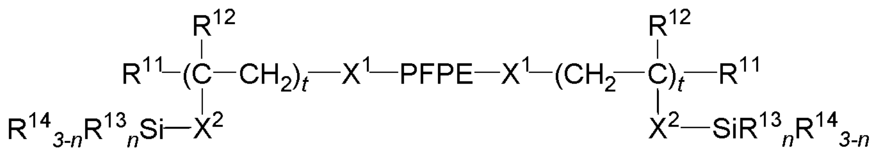
(A2)

wherein, each independently at each occurrence:

PFPE is $-(OC_3F_6)_d-$ wherein the repeating unit- $(OC_3F_6)$- has a branched structure, and *d* is an integer of 2-200;
Rf is $C_{1-16}$-alkyl optionally substituted by F;
$X^1$ is $-(R^{31})_{p'}-((X^b)_{l'})_{q'}-$; wherein, each independently at each occurrence,
$R^{31}$ is a single bond, $-(CH_2)_{s'}-$ optionally substituted by one or more F, or an o-, m-, or p-phenylene;
s' is an integer of 1-20;
$X^b$ is -O-, $-(OR^{35})_{n4}-$, -S-, o-, m- or p-phenylene, -C(O)O-, $-Si(R^{33})_2-$, $-(Si(R^{33})_2O)_{m'}-Si(R^{33})_2-$, $-CON(R^{34})-$, $-O-CON(R^{34})-$, $-N(R^{34})-$ or $-(CH_2)_{n'}-$,
wherein
$R^{33}$ is phenyl, a $C_{1-6}$-alkyl or $C_{1-6}$-alkoxy;
$R^{34}$ is H, phenyl or $C_{1-6}$-alkyl;
$R^{35}$ is $C_{1-6}$-alkylene;
n4 is an integer of 1-5;
m' is an integer of 1-100;
n' is an integer of 1-20;
l' is an integer of 1-10;
p' is 0 or 1;
q' is 0 or 1; at least one of p' and q' is 1, and the order of the repeating units in parentheses is arbitrary;
$R^{11}$ is H or halogen;
$R^{12}$ is H or $C_{1-20}$-alkyl;
$X^2$ is a single bond or a divalent organic group;
$R^{13}$ is OH or a hydrolyzable group;
$R^{14}$ is H or $C_{1-22}$-alkyl;
n is an integer of 1-3 for each $(-SiR^{13}{}_nR^{14}{}_{3-n})$ unit;
t is an integer of 2-10;
in the formulae (A1) and (A2), there are at least two $SiR^{13}$;

$$(Rf\text{-}PFPE)_{\beta'}\text{-}X^3\text{-}(SiR^a{}_3)_\beta \quad (B1)$$

$$(R^a{}_3Si)_\beta\text{-}X^3\text{-}PFPE\text{-}X^3\text{-}(SiR^a{}_3)_\beta \quad (B2)$$

wherein PFPE and Rf are as defined above and, each independently at each occurrence:

$X^3$ is a single bond or a 2- to 10-valent organic group;
$\beta$ is an integer of 1-9;
$\beta'$ is an integer of 1-9;
$R^a$ is $-Z^3-SiR^{72}{}_{q1}R^{73}{}_{r1}$; wherein, each independently at each occurrence,
$Z^3$ is a divalent organic group and does not include a group which forms a siloxane bond together with a Si atom;
$R^{72}$ is OH or a hydrolyzable group;
$R^{73}$ is H or $C_{1-20}$-alkyl;
*q1* is 2 or 3;

r1 is 0 or 1;

$(q1+r1) = 3$ in each ($-Z^3-SiR^{72}_{q1}R^{73}_{r1}$);

$$Rf-PFPE-X^5-CR^e_3 \quad (C1)$$

$$R^e_3C-X^5-PFPE-X^5-CR^e_3 \quad (C2)$$

wherein PFPE and Rf are as defined above, $X^5$ is as defined for $X^1$ in formulae (A1) and (A2) above and, each independently at each occurrence:

$R^e$ is $-Y-SiR^{85}_{n2}R^{86}_{3-n2}$;
Y is a divalent organic group;
$R^{85}$ is OH or a hydrolyzable group;
$R^{86}$ is H or $C_{1-20}$-alkyl; and
n2 is an integer of 1-3 for each ($-Y-SiR^{85}_{n2}R^{86}_{3-n2}$) unit,
with the proviso that a compound of the formula

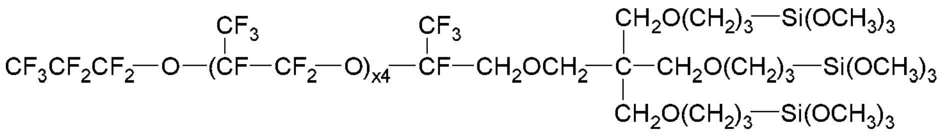

wherein x4 = 6 and is a mean value of the number of units, is excluded.

Also, the present invention provides (i) a surface-treating agent comprising the present compound, (ii) a pellet comprising the above surface-treating agent and (iii) an article comprising a base material and, on a surface of the base material, a layer formed of the above surface-treating agent.

Yet further, the present invention provides a compound of formula (c1-3) or (c2-3):

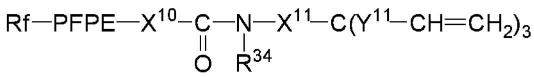

(c1-3)

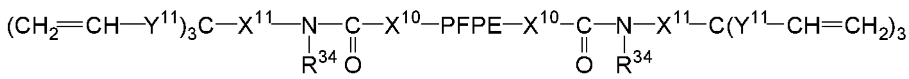

(c2-3)

wherein, each independently at each occurrence,

| | |
|---|---|
| Rf | is $C_{1-16}$-alkyl optionally substituted by F; |
| PFPE | is $(-OCF(CF_3)CF_2-)_d$ wherein d is an integer of 2-200; |
| $X^{10}$ | is a single bond or a divalent organic group; |
| $R^{34}$ | is H, phenyl or $C_{1-6}$-alkyl; |
| $X^{11}$ | is a single bond or a divalent organic group; and |
| $Y^{11}$ | is a single bond or a divalent organic group. |

Preferred embodiments of the invention are as defined in the appended dependent claims and/or in the following detailed description.

Advantageous Effects of Invention

**[0017]** [0007] According to the present disclosure, it is possible to provide a PFPE-containing silane compound that can contribute to the formation of a surface-treating layer having good UV durability.

Description of Embodiments

**[0018]** [0008] The "2- to 10-valent organic group" as used herein refers to a 2- to 10-valent group containing a carbon atom. Examples thereof include a 2- to 10-valent group obtained by removing 1-9 hydrogen atoms from a hydrocarbon group. Examples of the divalent organic group include a divalent group obtained by removing one hydrogen atom from a hydrocarbon group.

**[0019]** [0009] The "hydrocarbon group" as used herein refers to a group containing a carbon atom and a hydrogen atom which is obtained by removing a hydrogen atom from the molecule. Examples of the hydrocarbon group include, but are not limited to, a $C_{1-20}$-hydrocarbon group which is optionally substituted by one or more substituents, for example, an aliphatic hydrocarbon group and an aromatic hydrocarbon group. The "aliphatic hydrocarbon group" may be linear, branched or cyclic, and may be saturated or unsaturated. The hydrocarbon group may contain one or more ring structures. It is noted that the hydrocarbon group may have one or more N, O, S, Si, amide, sulfonyl, siloxane, carbonyl or carbonyloxy at the end or in the molecular chain thereof.

As used herein, examples of the substituent of the "hydrocarbon group" include a halogen atom; and one or more groups selected from $C_{1-6}$-alkyl, $C_{2-6}$-alkenyl, $C_{2-6}$-alkynyl, $C_{3-10}$-cycloalkyl, unsaturated $C_{3-10}$-cycloalkyl, 5- to 10-membered heterocyclyl, 5- to 10-membered unsaturated heterocyclyl, $C_{6-10}$-aryl, and 5- to 10-membered heteroaryl, which are optionally substituted by one or more halogen atoms.

The alkyl group and the phenyl group herein are optionally substituted unless otherwise specified. Examples of the substituent for such groups include, one or more groups selected from halogen, $C_{1-6}$-alkyl, $C_{2-6}$-alkenyl, and $C_{2-6}$-alkynyl.

The alkylene group herein refers to a group having a - $(C_\delta H_{2\delta})$- structure, and is optionally substituted, and may be linear or branched unless otherwise specified.

The present silane compound will be described below.

Formulae (A1) and (A2):

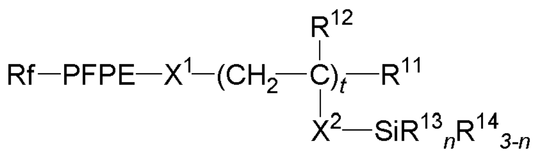

(A1)

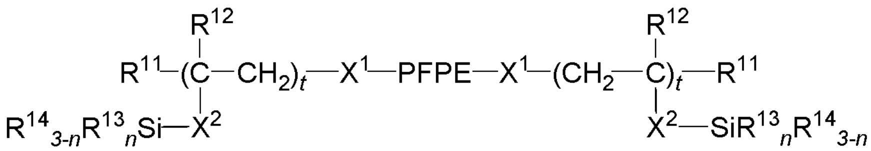

(A2)

In the formulae, Rf is $C_{1-16}$-alkyl optionally substituted by F. The $C_{1-16}$-alkyl in this group may be linear or branched, and preferably is linear or branched $C_{1-6}$-alkyl, in particular $C_{1-3}$-alkyl, and more preferably linear $C_{1-3}$-alkyl.

Rf is preferably $C_{1-16}$-alkyl substituted by F, more preferably $CF_2H$- ($C_{1-15}$-fluoroalkylene) or $C_{1-16}$-perfluoroalkyl, and still more preferably $C_{1-16}$-perfluoroalkyl.

The $C_{1-16}$-perfluoroalkyl may be linear or branched, and preferably is a linear or branched $C_{1-6}$-, in particular $C_{1-3}$-, perfluoroalkyl, and more preferably linear $C_{1-3}$-perfluoroalkyl, specifically $-CF_3$, $-CF_2CF_3$, or $-CF_2CF_2CF_3$.

In the formulae, PFPE is $-(OC_3F_6)_d$- wherein the repeating unit-$(OC_3F_6)$- has a branched structure, and d is an integer of 2-200 In formula (A1), in the PFPE, the oxygen atom at the left end of the formula is bonded to Rf, and the carbon atom at the right end of the formula is bonded to $X^1$. By having the PFPE having such a structure, the layer (for example, surface-treating layer) formed by using a PFPE-containing silane compound (or a surface-treating agent containing a PFPE-containing silane compound) can be further improved in UV durability, water-repellency, oil-repellency, antifouling property (for example, preventing adhesion of fouling such as fingerprints), chemical resistance, hydrolysis resistance, an effect of suppressing lubricity, high friction durability, heat resistance and moisture-proof property.

In the formulae, dis an integer of 2-200, preferably 5-200, more preferably 10-200.

Examples of $-(OC_3F_6)$- include $-(OCF(CF_3)CF_2)$- and - $(OCF_2CF(CF_3))$ -.

In the formulae, $R^{13}$ each independently hydroxyl or a hydrolyzable group.

As used herein, the "hydrolyzable group" refers to a group capable of undergoing a hydrolysis reaction, that

is, a group capable of being removed from the main backbone of compound by a hydrolysis reaction. Examples of the hydrolyzable group include -OR, -OCOR, -O-N=$CR_2$, -$NR_2$, -NHR, halogen (wherein R is optionally substituted $C_{1-4}$-alkyl), preferably -OR (i.e., alkoxy). Examples of R include non-substituted alkyl such as methyl, ethyl, propyl, isopropyl, n-butyl and isobutyl; and substituted alkyl such as chloromethyl. Among them, non-substituted alkyl is preferred, and methyl or ethyl is more preferred. The hydroxyl may be, but is not limited to, a group generated by hydrolysis of a hydrolyzable group.

In the formulae, $R^{14}$ each independently is H or $C_{1-22}$-alkyl, and preferably $C_{1-4}$-alkyl.

In the formulae, $R^{11}$ each independently is H or halogen. The halogen is preferably I, Cl or F, more preferably F.

In the formulae, $R^{12}$ each independently is H or $C_{1-20}$-alkyl. The alkyl is preferably $C_{1-6}$-alkyl, and examples thereof include methyl, ethyl and propyl.

In the formulae, n each independently is an integer of 1-3, and preferably 3 for each ($-SiR^{13}_nR^{14}_{3-n}$) unit. In other words, there are at least two $SiR^{13}$ in the formulae (A1) and (A2) .

Preferably, in the formulae (A1) and (A2), n is 2 or 3, and more preferably n is 3. In other words, the Si atom is preferably present as $-SiR^{13}_2R^{14}$ or $-SiR^{13}_3$ and more preferably as $-SiR^{13}_3$.

In the formulae, $X^1$ each independently is $-(R^{31})_{p'}-((X^b)_{l'})_{q'}-$, wherein, each independently at each occurrence,:

$R^{31}$ is a single bond, $-(CH_2)_{s'}-$ optionally substituted by one or more F, or o-, m-, or p-phenylene, and preferably $-(CH_2)_{s'}-$ optionally substituted by one or more F,
s' is an integer of 1-20, preferably 1-6, more preferably 1-3, and still more preferably 1 or 2,
$X^b$ is a group selected from -O-, $-(OR^{35})_{n4}-$, -S-, o-, m-, or p-phenylene, -C(O)O-, $-Si(R^{33})_2-$, $-(Si(R^{33})_2O)_{m'}-Si(R^{33})_2-$, $-CON(R^{34})-$, $-O-CON(R^{34})-$, $-N(R^{34})-$, and $-(CH_2)_{n'}-$,
$R^{33}$ is phenyl, $C_{1-6}$-alkyl or $C_{1-6}$-alkoxy, preferably phenyl or $C_{1-6}$-alkyl, and more preferably methyl,
$R^{34}$ is H, phenyl or $C_{1-6}$-alkyl, preferably H or $C_{1-6}$-alkyl group, more preferably H or methyl, and still more preferably H,
$R^{35}$ is $C_{1-6}$-alkylene, preferably $C_{1-3}$-alkylene,
n4 is an integer of 1-5, preferably 1-3, and more preferably 1,
m' is an integer of 1-100, preferably 1-20,
n' is an integer of 1-20, preferably 1-6, and more preferably 1-3,
l' is an integer of 1-10, preferably 1-5, and more preferably 1-3,
p' is 0 or 1,
q' is 0 or 1, and
at least one of p' and q' is 1, and the order of the repeating units in parentheses with p' or q' is arbitrary.

Here, $R^{31}$ and $X^b$ (typically, a hydrogen atom in $R^{31}$ and $X^b$) are optionally substituted by one or more substituents selected from F, $C_{1-3}$-alkyl and $C_{1-3}$-fluoroalkyl.

In one embodiment, l' is 1.

Examples of $X^1$ include the following structure:

$$-X^{10}-CON(R^{34})-X^{11}-,$$

$$-X^{10}-(OR^{35})_{n4}-X^{11}-,$$

and $C_{1-6}$-alkylene (preferably propylene) .

When $X^1$ has the above structure, hydrolysis resistance, friction durability, chemical resistance and moisture-proof property of the layer formed of the PFPE-containing silane compound is further improved.

$X^{10}$ is each independently a single bond or a divalent organic group, preferably a single bond, $-O-R^{36}-O-$, $-R^{36}-$, or o-, m-, or p-phenylene, and more preferably a single bond, $-O-R^{36}-O-$, or $-R^{36}-$.

In one embodiment, $X^{10}$ is a single bond or $-R^{36}-$, and more preferably a single bond.

$R^{36}$ is $C_{1-20}$-alkylene , preferably $C_{1-3}$-alkylene , and still more preferably $C_{1-2}$-alkylene , each optionally substituted by one or more F. The alkylene group may be linear or branched.

In one embodiment, the alkylene optionally substituted by one or more F in $X^{10}$ is, for example, $C_{1-3}$-perfluoroalkylene, $C_{1-3}$-fluoroalkylene substituted by one or more, or unsubstituted $C_{1-3}$-alkylene, and more specifically $C_{1-2}$-perfluoroalkylene, and $C_{1-2}$-fluoroalkylene substituted by one or more F, or unsubstituted $C_{1-2}$-alkylene.

$X^{11}$ is each independently a single bond, O, or a divalent organic group, and preferably a single bond, O, or $C_{1-20}$-alkylene optionally substituted by one or more F.

In one embodiment, $X^{11}$ is preferably a single bond or $C_{1-20}$-alkylene optionally substituted by one or more F, more preferably a single bond or unsubstituted $C_{1-6}$-alkylene, still more preferably a single bond or unsubstituted $C_{1-3}$-alkylene, and particularly preferably unsubstituted $C_{1-3}$-alkylene.

The alkylene optionally substituted by one or more F in $X^{11}$ is preferably $C_{1-6}$-alkylene, and more preferably

$C_{1-3}$-alkylene, each optionally substituted by one or more F. The alkylene group may be linear or branched.

More preferably, the alkylene optionally substituted by one or more F in $X^{11}$ is preferably unsubstituted $C_{1-6}$-alkylene, and more preferably unsubstituted $C_{1-3}$-alkylene.

$R^{34}$ each independently is H, phenyl or $C_{1-6}$-alkyl, preferably H or $C_{1-6}$-alkyl, more preferably H or methyl, and still more preferably H.

$R^{35}$ each independently is $C_{1-6}$-alkylene, and preferably $C_{1-3}$-alkylene.

n4 each independently is an integer of 1-5, preferably 1-3, and more preferably 1.

In one embodiment, $X^1$ is $-X^{10}$-CON($R^{34}$)-$X^{11}$- or $C_{1-6}$-alkylene (preferably propylene).

In the formula:

$R^{34}$ is as defined above, and preferably H or $C_{1-6}$-alkyl, more preferably H or methyl, and still more preferably H;
$X^{10}$ is as defined above, and preferably a single bond, -$R^{36}$-, or o-, m-, or p-phenylene, more preferably a single bond or -$R^{36}$-, and still more preferably a single bond;
$R^{36}$ is as defined above, and preferably $C_{1-20}$-alkylene, more preferably $C_{1-3}$-alkylene, and still more preferably $C_{1-2}$-alkylene, each optionally substituted by one or more F; and
$X^{11}$ is as defined above, and preferably a single bond or $C_{1-20}$-alkylene optionally substituted by one or more F, more preferably a single bond or unsubstituted $C_{1-6}$-alkylene, still more preferably a single bond or unsubstituted $C_{1-3}$-alkylene, and particularly preferably unsubstituted $C_{1-3}$-alkylene.

In the embodiment, $X^1$ is particularly preferably $-X^{10}$-CON ($R^{34}$)-$X^{11}$-. In the formula, $R^{34}$, $X^{10}$, and $X^{11}$ are as defined above.

In the embodiment, $X^{10}$ is particularly preferably a single bond and $X^{11}$ is particularly preferably $C_{1-3}$-alkylene, and $X^{11}$ is still more preferably unsubstituted $C_{1-3}$-alkylene.

In one embodiment, $X^{10}$ is a single bond and $X^{11}$ is $C_{1-3}$-alkylene, and preferably, $X^{11}$ is unsubstituted $C_{1-3}$ alkylene.

Specific examples of $X^1$ include:

$-CH_2OCH_2-$,

$-CH_2O(CH_2)_2-$,

$-CH_2O(CH_2)_3-$,

$-CH_2O(CH_2)_6-$,

$-CF_2-CH_2OCH_2-$,

$-CF_2-CH_2O(CH_2)_2-$,

$-CF_2-CH_2O(CH_2)_3-$,

$-CF_2-CH_2O(CH_2)_6-$,

$-O-CFHCF_2-O-CH_2OCH_2-$,

$-O-CFHCF_2-O-CH_2O(CH_2)_2-$,

$-O-CFHCF_2-O-CH_2O(CH_2)_3-$,

$-O-CFHCF_2-O-CH_2O(CH_2)_6-$,

$-CH_2OCF_2CHFOCF_2-$,

$-CH_2OCF_2CHFOCF_2CF_2-$,

$-CH_2OCF_2CHFOCF_2CF_2CF_2-$,

$-CH_2OCH_2CF_2CF_2OCF_2-$,

$-CH_2OCH_2CF_2CF_2OCF_2CF_2-$,

$-CH_2OCH_2CF_2CF_2OCF_2CF_2CF_2-$,

$-CH_2OCH_2CF_2CF_2OCF(CF_3)CF_2OCF_2-$,

$-CH_2OCH_2CF_2CF_2OCF(CF_3)CF_2OCF_2CF_2-$,

$-CH_2OCH_2CF_2CF_2OCF(CF_3)CF_2OCF_2CF_2CF_2-$,

$-CH_2OCH_2CHFCF_2OCF_2-$,

$-CH_2OCH_2CHFCF_2OCF_2CF_2-$,

$-CH_2OCH_2CHFCF_2OCF_2CF_2CF_2-$,

$-CH_2OCH_2CHFCF_2OCF(CF_3)CF_2OCF_2-$,

$-CH_2OCH_2CHFCF_2OCF(CF_3)CF_2OCF_2CF_2-$,

$-CH_2OCH_2CHFCF_2OCF(CF_3)CF_2OCF_2CF_2CF_2-$,

$-CH_2OCF_2CHFOCF_2CF_2CF_2-C(O)NH-CH_2-$,

$-CH_2-$,

$-(CH_2)_2-$,

$-(CH_2)_3-$,

$-(CH_2)_4-$,

$-(CH_2)_5-$,

$-(CH_2)_6-$,

$-CF_2-$,

$-(CF_2)_2-$,

$-CF_2-CH_2-$,

$-CF_2-(CH_2)_2-$,

$-CF_2-(CH_2)_3-$,

$-CF_2-(CH_2)_4-$,

$-CF_2-(CH_2)_5-$,

$-CF_2-(CH_2)_6-$,

-CONH-,

$-CONH-CH_2-$,

$-CONH-(CH_2)_2-$,

$-CONH-(CH_2)_3-$,

$-CONH-(CH_2)_6-$,

$-CF_2-CONH-$,

$-CF_2-CONH-CH_2-$,

$-CF_2-CONH-(CH_2)_2-$,

$-CF_2-CONH-(CH_2)_3-$,

$-CF_2-CONH-(CH_2)_6-$,

$-O-CFHCF_2-O-CONH-$,

$-O-CFHCF_2-O-CONH-CH_2-$,

$-O-CFHCF_2-O-CONH-(CH_2)_2-$,

$-O-CFHCF_2-O-CONH-(CH_2)_3-$,

$-O-CFHCF_2-O-CONH-(CH_2)_6-$,

$-CON(CH_3)-$,

$-CON(CH_3)-CH_2-$,

$-CON(CH_3)-(CH_2)_2-$,

$-CON(CH_3)-(CH_2)_3-$,

$-CON(CH_3)-(CH_2)_6-$,

$-CON(CH_3)-C_6H_4-$,

$-CON(Ph)-(CH_2)_3-$ (wherein Ph is phenyl),

$-CON(Ph)-(CH_2)_6-$ (wherein Ph is phenyl),

$-CF_2-CON(CH_3)-$,

$-CF_2-CON(CH_3)-CH_2-$,

$-CF_2-CON(CH_3)-(CH_2)_2-$,

$-CF_2-CON(CH_3)-(CH_2)_3-$,

$-CF_2-CON(CH_3)-(CH_2)_6-$,

$-CF_2-CON(CH_3)-C_6H_4-$,

$-CF_2-CON(Ph)-(CH_2)_3-$ (wherein Ph is phenyl),

$-CF_2-CON(Ph)-(CH_2)_6-$ (wherein Ph is phenyl),

$-CH_2-CON(CH_3)-CH_2-$,

$-CH_2-CON(CH_3)-(CH_2)_2-$,

$-CH_2-CON(CH_3)-(CH_2)_3-$,

$-CH_2O-CONH-(CH_2)_3-$,

$-CH_2O-CONH-(CH_2)_6-$,

$-OCH_2-$,

$-O(CH_2)_3-$,

$-OCFHCF_2-$,

$-O-CFHCF_2-O-$,

$-O-CFHCF_2-O-CH_2-$,

$-O-CFHCF_2-O-(CH_2)_2-$,

$-O-CFHCF_2-O-(CH_2)_3-$,

$-O-CFHCF_2-O-(CH_2)_6-$,

$-O-CFHCF_2-O-C_6H_4-$,

or

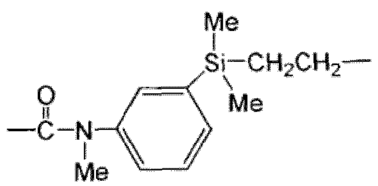

,

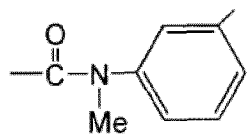

Examples of more preferred $X^1$ include:

$-O-CFHCF_2-O-CONH-$,

$-O-CFHCF_2-O-CONH-CH_2-$,

$-O-CFHCF_2-O-CONH-(CH_2)_2-$,

$-O-CFHCF_2-O-CONH-(CH_2)_3-$,

$-O-CFHCF_2-O-CONH-(CH_2)_6-$,

$-CONH-$,

$-CONH-CH_2-$,

$-CONH-(CH_2)_2-$,

$-CONH-(CH_2)_3-$,

$-CONH-(CH_2)_6-$,

$-CF_2-CONH-$,

$-CF_2-CONH-CH_2-$,

$-CF_2-CONH-(CH_2)_2-$,

$-CF_2-CONH-(CH_2)_3-$,

$-CF_2-CONH-(CH_2)_6-$,

$-O-CFHCF_2-O-CH_2OCH_2-$,

$-O-CFHCF_2-O-CH_2O(CH_2)_2-$,

$-O-CFHCF_2-O-CH_2O(CH_2)_3-$,

$-O-CFHCF_2-O-CH_2O(CH_2)_6-$,

$-CH_2OCH_2-$,

$-CH_2O(CH_2)_2-$,

$-CH_2O(CH_2)_3-$,

$-CH_2O(CH_2)_6-$,

$-CF_2-CH_2OCH_2-$,

$-CF_2-CH_2O(CH_2)_2-$,

$-CF_2-CH_2O(CH_2)_3-$,

$-CF_2-CH_2O(CH_2)_6-$,

$-O-CFHCF_2-O-$,

$-O-CFHCF_2-O-(CH_2)_2-$,

$-O-CFHCF_2-O-(CH_2)_3-$,

$-O-CFHCF_2-O-(CH_2)_6-$,

$-CH_2-$,

$-(CH_2)_2-$,

$-(CH_2)_3-$,

$-(CH_2)_6-$

$-CF_2-$,

$-(CF_2)_2-$,

$-CF_2-CH_2-$,

$-CF_2-(CH_2)_2-$,

$-CF_2-(CH_2)_3-$,

and

$-CF_2-(CH_2)_6-$.

In the formulae, $X^2$ each independently is a single bond or a divalent organic group. $X^2$ is preferably $C_{1-20}$-alkylene, and more preferably $-(CH_2)_u-$ (wherein u is an integer of 0-2).

In the formulae, t each independently is an integer of 2-10, preferably 2-6.

In a preferred embodiment, in formulae (A1) and (A2),

PFPE each independently is $-(OCF(CF_3)CF_2)_d-$ wherein d is an integer of 1-200, preferably 5-200, and more preferably 10-200;

$X^1$ each independently is

$-X^f-X^{10}-CON(R^{34})-X^{11}-$,
$-X^f-X^{10}-(OR^{35})_{n4}-X^{11}-$, or
$-X^f-(C_{1-6}$-alkylene, preferably propylene) ;

$X^f$ each independently is a single bond or $C_{1-6}$-alkylene, preferably a single bond or $C_{1-4}$-alkylene, and more preferably a single bond or $C_{1-2}$-alkylene (for example, methylene), wherein the hydrogen atom in $X^f$ is optionally substituted by one or more substituents selected from F, $C_{1-3}$-alkyl and $C_{1-3}$-fluoroalkyl, and preferably substituted;

$X^{10}$ each independently is a single bond, $-O-R^{36}-O-$, $-R^{36}-$, or o-, m-, or p-phenylene, preferably a single bond, $-O-R^{36}-O-$, or $-R^{36}-$, more preferably a single bond or $-R^{36}-$, and still more preferably a single bond;

$R^{36}$ is $C_{1-20}$-alkylene, preferably $C_{1-3}$-alkylene, and still more preferably $C_{1-2}$-alkylene, each optionally substituted by one or more F, wherein the alkylene group in $R^{36}$ may be linear or branched;

$R^{34}$ each independently is H, phenyl or $C_{1-6}$-alkyl, preferably H or $C_{1-6}$-alkyl, more preferably H or methyl, and still more preferably H;

$R^{35}$ each independently is $C_{1-6}$-alkylene, and preferably $C_{1-3}$-alkylene;

n4 each independently is an integer of 1-5, preferably 1-3, and more preferably 1;

$X^{11}$ each independently is a single bond, O, or $C_{1-20}$-alkylene optionally substituted by one or more F, preferably a single bond or unsubstituted $C_{1-6}$-alkylene, more preferably a single bond or unsubstituted $C_{1-3}$-alkylene, and particularly preferably unsubstituted $C_{1-3}$-alkylene;

$X^2$ is $-(CH_2)_u-$; and

u each independently is integer of 0-2.

In a preferred embodiment, the compounds (A1) and (A2) are compounds (A1') and (A2') wherein, in the formulae (A1) and (A2):

PFPE is $-(OCF(CF_3)CF_2)_d-$;

n is 2 or 3, and preferably 3;

$X^1$ each independently is $-X^{10}-CONH-X^{11}-$, $-X^{10}-(OR^{35})_{n4}-X1^1-$ ($R^{35}$ is $C_{1-3}$-alkylene; n4 is an integer of 1-5, preferably 1-3, and more preferably 1) or $C_{1-6}$-alkylene (preferably propylene);

$X^{10}$ each independently is a single bond, $-O-R^{36}-O-$, or $-R^{36}-$;

$R^{36}$ is $C_{1-3}$-alkylene optionally substituted by one or more F;

$X^{11}$ each independently is a single bond, O or $C_{1-3}$-alkylene;

$X^2$ is $-(CH_2)_u-$; and

u each independently is an integer of 0-2.

The number average molecular weight of the compounds (A1) and (A2) is, for example, 1,000-40,000, preferably 1,000-32,000, more preferably 1,000-20,000, and still more preferably 1,000-12,000. The number average molecular weight is a value measured by $^{19}F$-NMR and $^{1}H$-NMR.

In one embodiment, the compounds (A1) and (A2) have a number average molecular weight of 1,000-8,000, and preferably 1,000-4,000. By having such a number average molecular weight, a layer (for example, a surface-treating layer) formed by using the compound (or a surface-treating agent containing the compound) has particularly good slip

suppression effect and abrasion resistance.

In one embodiment, the compounds (A1) and (A2) have a weight average molecular weight Mw of 3,000 to < 6,000, and the molecular weight distribution (Mw/Mn) of the compounds is ≤ 1.2. The weight average molecular weight and the molecular weight distribution are determined, for example, based on gel filtration chromatography (GPC) measurement.

In a preferred embodiment, the compounds (A1) and (A2) are compounds (A1), and more preferably compounds (A1').

In one embodiment, the compounds (A1) and (A2) may be obtained, for example, by introducing a hydroxyl group in the end of a fluoropolyether derivative corresponding to the Rf-PFPE moiety as a raw material, and subjecting it to a Williamson reaction with a compound having an alkyl halide at the end thereof.

Formulae (B1) and (B2):

$$(\text{Rf-PFPE})_{\beta'}\text{-}X^3\text{-}(\text{SiR}^a{}_3)_\beta \qquad (B1)$$

$$(\text{R}^a{}_3\text{Si})_\beta\text{-}X^3\text{-PFPE-}X^3\text{-}(\text{SiR}^a{}_3)_\beta \qquad (B2)$$

In the formulae (B1) and (B2), Rf and PFPE are as defined for the formulae (A1) and (A2).

In the formulae, $X^3$ each independently is a single bond or a 2- to 10-valent organic group. $X^3$ in the compounds (B1) and (B2) is recognized to be a linker which connects a fluoropolyether moiety (i.e., an Rf-PFPE moiety or -PFPE-moiety) providing mainly good UV durability, water-repellency, oil-repellency, antifouling property (for example, preventing adhesion of fouling such as fingerprints), chemical resistance, hydrolysis resistance, an effect of suppressing lubricity, high friction durability, heat resistance and moisture-proof property and a silane moiety (specifically, - $SiR^a{}_{k1}R^b{}_{l1}R^c{}_{m1}$) providing an ability to bind to a base material. Therefore, $X^3$ may be a single bond or any organic group as long as the compounds (B1) and (B2) can stably exist. In the present specification, in the structure described as $X^3$, the left side of the structure is bonded to the group represented by PFPE, and the right side thereof is bonded to the group in parentheses with β.

In the formulae, β is an integer of 1-9, and β' is an integer of 1-9. These β and β' may vary depending on the valence number of $X^3$. In formula (B1), the sum of β and β' is the same as the valence number of $X^3$. For example, when $X^3$ is a 10-valent organic group, the sum of β and β' is 10, for example, β and β' may be 9 and 1, β and β' may be 5 and 5, or β and β' may be 1 and 9. When $X^3$ is a divalent organic group, β and β' are 1. In formula (B2), β is a value obtained by subtracting 1 from the valence number of $X^3$.

$X^3$ is preferably a 2- to 7-valent, more preferably 2-to 4--valent, and still more preferably a divalent organic group.

In one embodiment, $X^3$ is a 2- to 4-valent organic group, β is 1-3, and β' is 1.

In another embodiment, $X^3$ is a divalent organic group, β is 1, and β' is 1. In this case, the formulae (B1) and (B2) are represented by the formulae (B1') and (B2'):

$$\text{Rf-PFPE-}X^3\text{-SiR}^a{}_3 \qquad (B1')$$

$$\text{R}^a{}_3\text{Si-}X^3\text{-PFPE-}X^3\text{-SiR}^a{}_3 \qquad (B2')$$

Examples of $X^3$ include the same groups as those described for $X^1$.

Further examples of $X^3$ include the following groups:

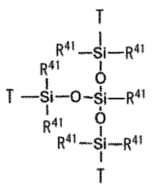
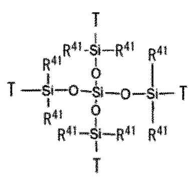
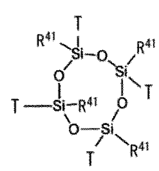
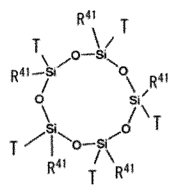

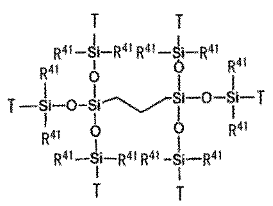
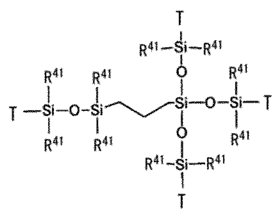

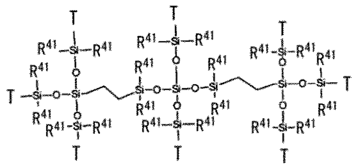

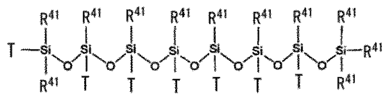

wherein:

$R^{41}$ is each independently a hydrogen atom, a phenyl group, an alkyl group having 1 to 6 carbon atoms, or a $C_{1-6}$ alkoxy group, and preferably a methyl group; and
in each $X^1$ group, some of T are the following group which binds to PFPE of the main backbone:

$-CH_2O(CH_2)_2-$,

$-CH_2O(CH_2)_3-$,

$-CF_2O(CH_2)_3-$,

$-CH_2-$,

$-(CH_2)_2-$,

$-(CH_2)_3-$,

$-(CH_2)_4-$,

$-CONH-(CH_2)_3-$,

$-CON(CH_3)-(CH_2)_3-$,

$-CON(Ph)-(CH_2)_3-$ (wherein Ph is phenyl), or

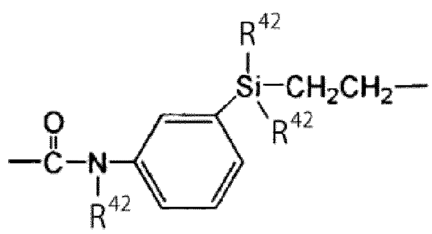

wherein $R^{42}$ each independently represents a hydrogen atom, a $C_{1-6}$ alkyl group, or a $C_{1-6}$ alkoxy group, preferably a methyl group or a methoxy group, and more preferably a methyl group,
some of the other T are $-(CH_2)_{n''}-$ (wherein n" is an integer of 2 to 6) bonded to the group opposite to PFPE which is a main backbone, and the others T are each independently a methyl group, a phenyl group, or a $C_{1-6}$ alkoxy or a radical scavenging group or a UV absorbing group, if present.

The radical scavenging group is not limited as long as it can trap a radical generated by light irradiation, and examples thereof include a residue of benzophenones, benzotriazoles, benzoic esters, phenyl salicylates, crotonic acids, malonic esters, organoacrylates, hindered amines, hindered phenols, or triazines.
The UV absorbing group is not limited as long as it can absorb ultraviolet rays, and examples thereof include residues of benzotriazoles, hydroxybenzophenones, esters of substituted and unsubstituted benzoic acid or salicylic acid compounds, acrylate or alkoxycinnamates, oxamides, oxanilides, benzoxazinones, and benzoxazoles.
In a preferred embodiment, examples of the radical scavenging group or the UV absorbing group include:

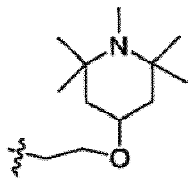, 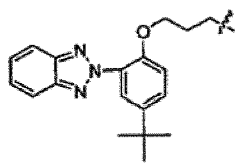 or 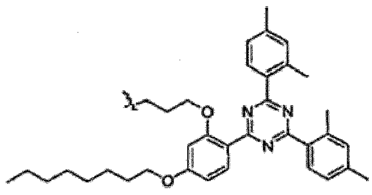

In this embodiment, $X^1$ (and $X^3$ and $X^5$ described below) may be a 3 to 10 valent organic group.

Examples of preferred $X^3$ include:

$-X^{10}-CON(R^{34})-X^{11}-$,

$-X^{10}-(OR^{35})_{n4}-X^{11}-$,

and

$C_{1-6}$-alkylene (preferably propylene). In the formulae, $R^{34}$, $R^{35}$, $X^{10}$, $X^{11}$, and n4 are as defined above.

Specific examples of particularly preferred $X^3$ include:

$-O-CFHCF_2-O-CONH-$,

$-O-CFHCF_2-O-CONH-CH_2-$,

$-O-CFHCF_2-O-CONH-(CH_2)_2-$,

$-O-CFHCF_2-O-CONH-(CH_2)_3-$,

$-O-CFHCF_2-O-CONH-(CH_2)_6-$,

$-CONH-$,

$-CONH-CH_2-$,

$-CONH-(CH_2)_2-$,

$-CONH-(CH_2)_3-$,

$-CONH-(CH_2)_6-$,

$-CF_2CONH-$,

$-CF_2CONHCH_2-$,

$-CF_2CONH(CH_2)_2-$,

$-CF_2CONH(CH_2)_3-$,

$-CF_2CONH(CH_2)_6-$,

$-O-CFHCF_2-O-CH_2OCH_2-$,

$-O-CFHCF_2-O-CH_2O(CH_2)_2-$,

$-O-CFHCF_2-O-CH_2O(CH_2)_3-$,

$-O-CFHCF_2-O-CH_2O(CH_2)_6-$,

$-CH_2OCH_2-$,

$-CH_2O(CH_2)_2-$,

$-CH_2O(CH_2)_3-$,

$-CH_2O(CH_2)_6-$,

$-CF_2-CH_2-O-CH_2-$,

$-CF_2-CH_2-O-(CH_2)_2-$,

$-CF_2-CH_2-O-(CH_2)_3-$,

$-CF_2-CH_2-O-(CH_2)_6-$,

$-O-CFHCF_2-O-$,

$-O-CFHCF_2-O-(CH_2)_2-$,

$-O-CFHCF_2-O-(CH_2)_3-$,

$-O-CFHCF_2-O-(CH_2)_6-$,

$-CH_2-$,

$-(CH_2)_2-$,

$-(CH_2)_3-$,

$-(CH_2)_6-$,

$-CF_2-$,

$-(CF_2)_2-$,

$-CF_2-CH_2-$,

$-CF_2-(CH_2)_2-$,

$-CF_2-(CH_2)_3-$,

$-CF_2-(CH_2)_4-$,

$-CF_2-(CH_2)_5-$,

and

$-CF_2-(CH_2)_6-$.

In a preferred embodiment, $X^3$ is

$-X^{10}-CON (R^{34})-X^{11}-$,

or
$C_{1-6}$-alkylene (preferably propylene) .
In the formula:

$R^{34}$ is as defined above, and preferably H or $C_{1-6}$-alkyl, more preferably H or methyl, and still more preferably H;

$X^{10}$ is as defined above, and preferably a single bond, $-R^{36}-$, or o-, m-, or p-phenylene, more preferably a single bond or $-R^{36}-$, and still more preferably a single bond;

$R^{36}$ is as defined above, and preferably $C_{1-20}$-alkylene, more preferably $C_{1-3}$-alkylene, and still more preferably $C_{1-2}$-alkylene, each optionally substituted by one or more F; and

$X^{11}$ is as defined above, and preferably a single bond or $C_{1-20}$-alkylene optionally substituted by one or more F, more preferably a single bond or unsubstituted $C_{1-6}$-alkylene, still more preferably a single bond or - unsubstituted $C_{1-3}$-alkylene, and particularly preferably unsubstituted $C_{1-3}$-alkylene.

In the embodiment, $X^3$ is particularly preferably $-X^{10}-CON(R^{34})-X^{11}-$. In the formula, $R^{34}$, $X^{10}$, and $X^{11}$ are as defined above.

In the embodiment, $X^{10}$ is particularly preferably a single bond and $X^{11}$ is particularly preferably $C_{1-3}$-alkylene, and $X^{11}$ is still more preferably unsubstituted $C_{1-3}$-alkylene.

In one embodiment, examples of $X^3$ include the following structure:

$-X^f-X^{10}-CON(R^{34})-X^{11}-$,

$-X^f-X^{10}-(OR^{35})_{n4}-X^{11}-$,

and

$-X^f-(C_{1-6}$-alkylene, preferably propylene) .

In the formula, $X^f$, $X^{10}$, $R^{34}$, $X^{11}$, $R^{35}$, and n4 are as defined above.

In the formula, $R^a$ each independently is $-Z^3-SiR^{71}_{p1}R^{72}_{q1}R^{73}_{r1}$.

In the formula, $Z^3$ each independently is a divalent organic group and does not include a group which forms a siloxane bond together with a Si atom

$Z^3$ is preferably $C_{1-6}$-alkylene, $-(CH_2)_g-O-(CH_2)_h-$(wherein g is an integer of 1-6, h is an integer of 1-6) or -phenylene-$(CH_2)_i$- (wherein i is an integer of 0-6), more preferably $C_{1-6}$-alkylene, and particularly preferably $C_{1-3}$-alkylene. These groups are optionally substituted by, for example, one or more substituents selected from F, $C_{1-6}$-alkyl, $C_{2-6}$ alkenyl and $C_{2-6}$-alkynyl. From the viewpoint of particularly good UV durability, $Z^3$ is more preferably linear or branched alkylene, and still more preferably linear alkylene. The number of carbon atoms constituting the alkylene group for $Z^3$ is preferably 1-6, more preferably 1-3. The alkylene group is as described above.

In one embodiment, $Z^3$ may be $C_{1-6}$-alkylene group or - phenylene-$(CH_2)_i$-. When $Z^3$ is a group as described above, light resistance, especially ultraviolet resistance can be further increased. In the formula, i is an integer of 0-6.

In the embodiment, $Z^3$ is preferably $C_{1-6}$-alkylene, and more preferably $C_{1-3}$-alkylene.

In one embodiment,

$X^3$ is preferably $-X^{10}-CON(R^{34})-X^{11}-$ or $C_{1-6}$-alkylene (preferably propylene), and more preferably $-X^{10}-CON(R^{34})-X^{11}-$; and

$Z^3$ is preferably $C_{1-6}$-alkylene or -phenylene-$(CH_2)_i$-, more preferably $C_{1-6}$-alkylene, and still more preferably $C_{1-3}$-alkylene.

$R^{34}$, $X^{10}$, $X^{11}$, and i are as defined above.

In one embodiment,

$X^3$ is preferably $-X^{10}-CON(R^{34})-X^{11}-$ or $C_{1-6}$-alkylene (preferably propylene), and more preferably $-X^{10}-CON(R^{34})-X^{11}-$; and

$Z^3$ is preferably $C_{1-6}$-alkylene or -phenylene-$(CH_2)_i$-, more preferably $C_{1-6}$-alkylene, and still more preferably $C_{1-3}$-alkylene.

In the formula:

$R^{34}$ is H or $C_{1-6}$-alkyl, preferably H or methyl, and more preferably H;

$X^{10}$ is a single bond, $-R^{36}-$, or o-, m-, or p-phenylene, preferably a single bond or $-R^{36}-$, and more preferably a single bond;

$R^{36}$ is $C_{1-20}$-alkylene, preferably $C_{1-3}$-alkylene gr, and more preferably $C_{1-2}$-alkylene, each optionally substituted by one or more F;

$X^{11}$ is a single bond or $C_{1-20}$-alkylene optionally substituted by one or more F, preferably a single bond or unsubstituted $C_{1-6}$-alkylene, more preferably a single bond or unsubstituted $C_{1-3}$-alkylene, and particularly preferably unsubstituted $C_{1-3}$-alkylene; and

i is an integer of 0-6.

In the embodiment, $X^{10}$ is particularly preferably a single bond and $X^{11}$ is particularly preferably $C_{1-3}$-alkylene, and $X^{11}$ is still more preferably unsubstituted $C_{1-3}$-alkylene. $X^3$ is more preferably $-X^{10}$-CON $(R^{34})$-$X^{11}$-.

In one embodiment,

$X^3$ is $-X^f-X^{10}-(OR^{35})_{n4}-X^{11}-$;

$Z^3$ is $C_{1-6}$-alkylene, and still more preferably $C_{1-3}$-alkylene. In the formula, $X^f$, $X^{10}$, $X^{11}$, $R^{35}$, and n4 are as defined above.

In the formulae, $R^{72}$ each independently is OH or a hydrolyzable group. The "hydrolyzable group" is as defined above.

$R^{72}$ is preferably a hydrolyzable group, and more preferably -OR (wherein R is optionally substituted $C_{1-3}$-alkyl, and more preferably methyl).

In the formulae, $R^{73}$ each independently is H or $C_{1-20}$-alkyl. The alkyl is preferably $C_{1-6}$-alkyl, and still more preferably methyl.

In one embodiment, $R^{73}$ is $C_{1-20}$-alkyl as defined above.

In formulae (B1) and (B2), there is $-SiR^{72}_2$ (specifically, $-SiR^{72}_2R^{73}$ or $-SiR^{72}_3$).

In the formulae, the unit $(-Z^3-SiR^{72}_{q1}R^{73}_{r1})$ is preferably $(-Z^3-SiR^{72}_3)$. In a still more preferred embodiment, all ends in $R^a$ may be - $-Si(-Z^3-SiR^{72}_3)_3$. [

Examples of preferable PFPE-containing silane compounds of formula (B1) include the following compounds. In the following, Rf is as defined above and d is 5-40.

$Rf-(OCF(CF_3)CF_2)_d-OCF(CF_3)-CH_2CH_2CH_2-$ $Si(CH_2CH_2SiCH_3(OCH_3)_2)_3$,

$Rf-(OCF(CF_3)CF_2)_d-OCF(CF_3)-CH_2CH_2CH_2-$ $Si(CH_2CH_2CH_2SiCH_3(OCH_3)_2)_3$,

$Rf-(OCF(CF_3)CF_2)_d-OCF(CF_3)-CH_2OCH_2CH_2CH_2-$ $Si(CH_2CH_2SiCH_3(OCH_3)_2)_3$,

$Rf-(OCF(CF_3)CF_2)_d-OCF(CF_3)-CH_2OCH_2CH_2CH_2-$ $Si(CH_2CH_2CH_2SiCH_3(OCH_3)_2)_3$,

$Rf-(OCF(CF_3)CF_2)_d-OCF(CF_3)-CONH-CH_2-$ $Si(CH_2CH_2SiCH_3(OCH_3)_2)_3$,

$Rf-(OCF(CF_3)CF_2)_d-OCF(CF_3)-CONH-CH_2-$ $Si(CH_2CH_2CH_2SiCH_3(OCH_3)_2)_3$,

$Rf-(OCF(CF_3)CF_2)_d-OCF(CF_3)-CONH-CH_2CH_2CH_2-$ $Si(CH_2CH_2SiCH_3(OCH_3)_2)_3$,

$Rf-(OCF(CF_3)CF_2)_d-OCF(CF_3)-CONH-CH_2CH_2CH_2-$ $Si(CH_2CH_2CH_2SiCH_3(OCH_3)_2)_3$,

$Rf-(OCF(CF_3)CF_2)_d-OCF(CF_3)-CH_2CH_2CH_2-Si(CH_2CH_2Si(OCH_3)_3$,

$Rf-(OCF(CF_3)CF_2)_d-OCF(CF_3)-CH_2CH_2CH_2-$ $Si(CH_2CH_2CH_2Si(OCH_3)_3)_3$,

$Rf-(OCF(CF_3)CF_2)_d-OCF(CF_3)-CH_2OCH_2CH_2CH_2-$ $Si(CH_2CH_2Si(OCH_3)_3)_3$,

$Rf-(OCF(CF_3)CF_2)_d-OCF(CF_3)-CH_2OCH_2CH_2CH_2-$ $Si(CH_2CH_2CH_2Si(OCH_3)_3)_3$,

$Rf-(OCF(CF_3)CF_2)_d-OCF(CF_3)-CONH-CH_2-Si(CH_2CH_2Si$ $(OCH_3)_3)_3$,

$Rf-(OCF(CF_3)CF_2)_d-OCF(CF_3)-CONH-CH_2-$ $Si(CH_2CH_2CH_2Si(OCH_3)_3)_3$,

$Rf-(OCF(CF_3)CF_2)_d-OCF(CF_3)-CONH-CH_2CH_2CH_2-$ $Si(CH_2CH_2Si(OCH_3)_3)_3$,

$Rf-(OCF(CF_3)CF_2)_d-OCF(CF_3)-CONH-CH_2CH_2CH_2-$ $Si(CH_2CH_2CH_2Si(OCH_3)_3)_3$

The number average molecular weight of the compounds (B1) and (B2) may be, for example, $5 \times 10^2$ to $1 \times 10^5$. Within this range, the number average molecular weight is preferably 1,000-30,000, more preferably 1,000-12,000, and still more preferably 1,000-6,000. In the present disclosure, the number average molecular weight is a value measured

by $^{19}F$-NMR.

In a preferred embodiment, in the formulae (B1) and (B2),

PFPE each independently is $-(OCF(CF_3)CF_2)_d-$, wherein d is an integer of 1-200, preferably 5-200, and more preferably 10-200;
$X^3$ each independently is $-X^f-X^{10}-CON(R^{34})-X^{11}-$, $-X^f-X^{10}-(OR^{35})_{n4}-X^{11}-$, or $-X^f-$ ($C_{1-6}$-alkylene, preferably propylene), and preferably $-X^f-X^{10}-CON(R^{34})-X^{11}-$;
$X^f$ each independently is a single bond or $C_{1-6}$-alkylene, preferably a single bond or $C_{1-4}$-alkylene, and more preferably a single bond or $C_{1-2}$-alkylene (for example, methylene), wherein the hydrogen atom in $X^f$ is optionally substituted by one or more substituents selected from F, a $C_{1-3}$-alkyl and $C_{1-3}$-fluoroalkyl, and preferably substituted;
$X^{10}$ each independently is a single bond, $-O-R^{36}-O-$, $-R^{36}-$, or o-, m-, or p-phenylene, preferably a single bond, $-O-R^{36}-O-$, or $-R^{36}-$, more preferably a single bond or $-R^{36}-$, and still more preferably a single bond;
$R^{36}$ is $C_{1-20}$-alkylene, preferably $C_{1-3}$-alkylene, , and still more preferably $C_{1-2}$-alkylene; each optionally substituted by one or more F, wherein the alkylene group in $R^{36}$ may be linear or branched;
$X^{11}$ each independently is a single bond, O, or $C_{1-20}$-alkylene optionally substituted by one or more F, preferably a single bond or unsubstituted $C_{1-6}$-alkylene, more preferably a single bond or unsubstituted $C_{1-3}$-alkylene, and particularly preferably unsubstituted $C_{1-3}$-alkylene;
$R^{34}$ each independently is H, phenyl or $C_{1-6}$-alkyl, preferably H or $C_{1-6}$-alkyl, more preferably H or methyl, and still more preferably H;
$R^{35}$ each independently is $C_{1-6}$-alkylene, and preferably $C_{1-3}$-alkylene;
n4 each independently is an integer of 1-5, preferably 1-3, and more preferably 1;
$Z^3$ is preferably $C_{1-6}$-alkylene or $-phenylene-(CH_2)_i-$, more preferably $C_{1-6}$-alkylene, and still more preferably $C_{1-3}$-alkylene; and
i is an integer of 0-6.

In one embodiment, the compounds (B1) and (B2) have a number average molecular weight of 1,000-8,000, and preferably 1,000-4,000. By having such a number average molecular weight, a layer (for example, a surface-treating layer) formed by using the compound (or a surface-treating agent containing the compound) can be further improved in slip suppression effect and friction durability.

In a preferred embodiment, the compounds (B1) and (B2) are compounds (B1).

In one embodiment, the compounds (B1) and (B2) may be prepared, for example, by introducing a hydroxyl group in the end of a fluoropolyether derivative corresponding to the Rf-PFPE moiety as a raw material, followed by further introducing a group having an unsaturated bond in the end thereof, and reacting the group having an unsaturated bond with a silyl derivative having a halogen atom, further introducing a hydroxyl group in the end and then a group having an unsaturated bond into the silyl group, and finally reacting the group having an unsaturated bond with a silyl derivative. For example, it may be synthesized as described in WO 2014/069592.

Formulae (C1) and (C2):

$$Rf-PFPE-X^5-CR^e_3 \qquad (C1)$$

$$R^e_3C-X^5-PFPE-X^5-CR^e_3 \qquad (C2)$$

In the formulae (C1) and (C2), Rf and PFPE are as defined for formulae (A1) and (A2), and $X^5$ is as defined for $X^1$ in formulae (A1) and (A2). Here, the "formula (A1)" is to be read as "formula (C1)" and the "$X^1$ group" is to be read as "$X^5$ group".

Examples of $X^5$ include the same group as those described for $X^1$.

Examples of preferred $X^5$ include:

$$-X^{10}-CON(R^{34})-X^{11}-,$$

$$-X^{10}-(OR^{35})_{n4}-X^{11}-,$$

and
$C_{1-6}$-alkylene (preferably propylene). In the formula, $R^{34}$, $R^{35}$, $X^{10}$, $X^{11}$, and n4 are as defined above.

In one embodiment $X^5$ is a single bond.

In one embodiment, examples of $X^5$ include the following structure:

$$-X^f-X^{10}-CON(R^{34})-X^{11}-,$$

$-X^f-X^{10}-(OR^{35})_{n4}-X^{11}-$,

and
$-X^f-$ ($C_{1-6}$-alkylene, preferably propylene) .
In the formula, $X^f$, $X^{10}$, $R^{34}$, $X^{11}$, $R^{35}$, and n4 are as defined above.
Examples of particularly preferred $X^5$ include:
a single bond;

$-O-CFHCF_2-O-CONH-$,

$-O-CFHCF_2-O-CONH-CH_2-$,

$-O-CFHCF_2-O-CONH-(CH_2)_2-$,

$-O-CFHCF_2-O-CONH-(CH_2)_3-$,

$-O-CFHCF_2-O-CONH-(CH_2)_6-$,

$-CONH-$,

$-CONH-CH_2-$,

$-CONH-(CH_2)_2-$,

$-CONH-(CH_2)_3-$,

$-CONH-(CH_2)_6-$,

$-CF_2-CONH-$,

$-CF_2-CONH-CH_2-$,

$-CF_2-CONH-(CH_2)_2-$,

$-CF_2-CONH-(CH_2)_3-$,

$-CF_2-CONH-(CH_2)_6-$,

$-O-CFHCF_2-O-CH_2OCH_2-$,

$-O-CFHCF_2-O-CH_2O(CH_2)_2-$,

$-O-CFHCF_2-O-CH_2O(CH_2)_3-$,

$-O-CFHCF_2-O-CH_2O(CH_2)_6-$,

$-CH_2OCH_2-$,

$-CH_2O(CH_2)_2-$,

$-CH_2O(CH_2)_3-$,

$-CH_2O(CH_2)_6-$,

$-CF_2-CH_2OCH_2-$,

$-CF_2-CH_2O(CH_2)_2-$,

$-CF_2-CH_2O(CH_2)_3-$,

$-CF_2-CH_2O(CH_2)_6-$,

$-O-CFHCF_2-O-$,

$-O-CFHCF_2-O-(CH_2)_2-$,

$-O-CFHCF_2-O-(CH_2)_3-$,

$-O-CFHCF_2-O-(CH_2)_6-$,

$-CH_2-$,

$-(CH_2)_2-$,

$-(CH_2)_3-$,

$-(CH_2)_6-$

$-CF_2-$,

$-(CF2)_2-$,

$-CF_2-CH_2-$,

$-CF_2-(CH_2)_2-$,

$-CF_2-(CH_2)_3-$,

$-CF_2-(CH_2)_4-$,

$-CF_2-(CH_2)_5-$,

and

$-CF_2-(CH_2)_6-$.

In a preferred embodiment, $X^5$ is

$-X^{10}-CON(R^{34})-X^{11}-$,

or

$C_{1-6}$-alkylene (preferably propylene) .

In the formula:

$R^{34}$ is as defined above, and is preferably H or $C_{1-6}$-alkyl, more preferably H or methyl, and still more preferably H;
$X^{10}$ is as defined above, and preferably a single bond, $-R^{36}-$, or o-, m-, or p-phenylene, more preferably a single bond or $-R^{36}-$, and still more preferably a single bond;
$R^{36}$ is as defined above, and preferably $C_{1-20}$-alkylene, more preferably $C_{1-3}$-alkylene, and still more preferably $C_{1-2}$-alkylene, each optionally substituted by one or more F; and
$X^{11}$ is as defined above, and preferably a single bond or $C_{1-20}$-alkylene optionally substituted by one or more F, more preferably a single bond or unsubstituted $C_{1-6}$-alkylene, still more preferably a single bond or unsubstituted $C_{1-3}$-alkylene, and particularly preferably unsubstituted $C_{1-3}$-alkylene.

In the embodiment, $X^5$ is particularly preferably $-X^{10}-CON(R^{34})-X^{11}-$ . In the formula, $R^{34}$, $X^{10}$, and $X^{11}$ are as defined above.

In the embodiment, $X^{10}$ is particularly preferably a single bond and $X^{11}$ is particularly preferably $C_{1-3}$-alkylene, and $X^{11}$ is still more preferably unsubstituted $C_{1-3}$-alkylene.

Y each independently is a divalent organic group.

In a preferred embodiment, Y is $C_{1-6}$-alkylene, $-(CH_2)_{g'}-O-(CH_2)_{h'}-$ (wherein g' is an integer of 0-6 (e.g. 1-6), and h' is an integer of 0-6 (e.g. 1-6) or -phenylene-$(CH_2)_{i'}$-(wherein i' is an integer of 0-6. These groups are optionally substituted by, for example, one or more substituents selected from F, $C_{1-6}$-alkyl, $C_{2-6}$-alkenyl and $C_{2-6}$-alkynyl.

In one embodiment, Y may be $C_{1-6}$-alkylene or - phenylene- $(CH_2)_{i'}$-. When Y is a group as described above, light resistance, especially UV resistance can be further increased.

Y is preferably $C_{1-6}$-alkylene group, and more preferably $C_{2-3}$-alkylene.

In one embodiment,

$X^5$ is preferably $-X^{10}-CON(R^{34})-X^{11}-$ or $C_{1-6}$-alkylene (preferably propylene), and more preferably $-X^{10}-CON(R^{34})-X^{11}-$; and
Y is preferably $C_{1-6}$-alkylene or -phenylene- $(CH_2)_{i'}$-, and more preferably $C_{1-6}$-alkylene.
$R^{34}$, $X^{10}$, $X^{11}$ and i' are as defined above.

In one embodiment,

$X^5$ is preferably $-X^{10}-CON(R^{34})-X^{11}-$ or $C_{1-6}$-alkylene (preferably propylene), and more preferably $-X^{10}-CON(R^{34})-X^{11}-$; and
Y is preferably $C_{1-6}$-alkylene or -phenylene-$(CH_2)_{i'}$-, more preferably $C_{1-6}$-alkylene, and still more preferably $-C_{2-3}$-alkylene.

In the formula:

$R^{34}$ is H or $C_{1-6}$-alkyl, preferably H or methyl, and more preferably H;
$X^{10}$ is a single bond, $-R^{36}-$, or o-, m-, or p-phenylene, preferably a single bond or $-R^{36}-$, and more preferably a single bond;
$R^{36}$ is $C_{1-20}$-alkylene, preferably $C_{1-3}$-alkylene s, and more preferably $C_{1-2}$-alkylene, each optionally substituted by one or more F;
$X^{11}$ is a single bond or $C_{1-20}$-alkyleneoptionally substituted by one or more F, preferably a single bond or unsubstituted $C_{1-6}$-alkylene, more preferably a single bond or unsubstituted $C_{1-3}$-alkylene, and particularly preferably unsubstituted $C_{1-3}$-alkylene; and
i' is an integer of 0-6.

In the embodiment, $X^{10}$ is particularly preferably a single bond and $X^{11}$ is particularly preferably $C_{1-3}$-alkylene, and $X^{11}$ is still more preferably unsubstituted $C_{1-3}$-alkylene. More preferably, $X^5$ is $-X^{10}-CON(R^{34})-X^{11}-$.

$R^{85}$ each independently is OH or a hydrolyzable group. The "hydrolyzable group" is as defined above.

$R^{85}$ is preferably -OR (wherein R is optionally substituted $C_{1-3}$-alkyl, more preferably methyl or ethyl, and particularly methyl).

$R^{86}$ each independently is H or $C_{1-20}$-alkyl group. The alkyl is preferably $C_{1-6}$-alkyl, and still more preferably methyl.

n2 independently is an integer of 1-3, more preferably 2 or 3, and particularly preferably 3 for each $(-Y-SiR^{85}_{n2}R^{86}_{3-n2})$ unit.

In the formula, $R^e$ each independently is $-Y-SiR^{85}_{n2}R^{86}_{3-n2}$.

Preferably, in formulae (C1) and (C2), there are at least two groups represented by $-Y-SiR^{85}_{n2}R^{86}_{3-n2}$ in which n2 is $\geq 2$. That is, preferably, there are at least two groups represented by $-Y-SiR^{85}_2R^{86}$ or $-Y-SiR^{85}_3$.

In formulae (C1) and (C2), the unit $(-Y-SiR^{85}_{n2}R^{86}_{3-n2})$ is preferably $(-Y-SiR^{85}_2R^{86})$ or $(-Y-SiR^{85}_3)$, and more preferably $(-Y-SiR^{85}_3)$.

Examples of the PFPE-containing silane compound of the preferable formula (C1) include the following compounds. In the following, Rf is as defined above (for example, $CF_3CF_2CF_2$), and d is 5-40.

$$Rf-(OCF(CF_3)CF_2)_dOCF(CF_3)CONHCH_2C(CH_3)(CH_2CH_2CH_2Si(OCH_3)_3)_2,$$

$$Rf-(OCF(CF_3)CF_2)_dOCF(CF_3)CONHCH_2C(CH_2CH_2CH_2Si(OCH_3)_3)_3,$$

$$Rf-(OCF(CF_3)CF_2)_dOCF(CF_3)CH_2OCH_2C(CH_3)(CH_2CH_2CH_2Si(OCH_3)_3)_2,$$

$Rf-(OCF(CF_3)CF_2)_dOCF(CF_3)CH_2OCH_2C(CH_2CH_2CH_2Si(OCH_3)_3)_3$,

$Rf-(OCF(CF_3)CF_2)_dOCF(CF_3)CH_2OCH_2C(CH_3)(CH_2OCH_2CH_2CH_2Si(OCH_3)_3)_2$,

$Rf-(OCF(CF_3)CF_2)_dOCF(CF_3)CH_2OCH_2C(CH_2OCH_2CH_2CH_2Si(OCH_3)_3)_3$,

$Rf-(OCF(CF_3)CF_2)_dOCF(CF_3)-CH_2CH_2CH_2-$

$C(CH_3)(CH_2CH_2CH_2Si(OCH_3)_3)_2$,

$Rf-(OCF(CF_3)CF_2)_dOCF(CF_3)-CH_2CH_2CH_2-C(CH_2CH_2CH_2Si(OCH_3)_3)_3$

The number average molecular weight of the compounds (C1) and (C2) may be, for example, $5 \times 10^2$ to $1 \times 10^5$. Within this range, the number average molecular weight is preferably 1,000-30,000, more preferably 1,000-12,000, and still more preferably 1,000-6,000. In the present disclosure, the number average molecular weight is a value measured by $^{19}$F-NMR.

In one embodiment, the compounds (C1) and (C2) have a number average molecular weight of 1,000-8,000, and preferably 1,000-4,000. By having such a number average molecular weight, a layer (for example, a surface-treating layer) formed by using the compound (or a surface-treating agent containing the compound) can be further improved in slip suppression effect and abrasion resistance.

In one embodiment, PFPE each independently is - $(OCF(CF_3)CF_2)_d$- wherein d is an integer of 1-200, preferably 5-200, and more preferably 10-200;

$X^5$ is a single bond, -$X^f$-$X^{10}$-CON($R^{34}$)-$X^{11}$-, -$X^f$-$X^{10}$-$(OR^{35})_{n4}$-$X^{11}$-, or -$X^f$- ($C_{1-6}$-alkylene, preferably propylene);
$X^f$ each independently is a single bond or $C_{1-6}$-alkylene , preferably a single bond or $C_{1-4}$-alkylene, and more preferably a single bond or $C_{1-2}$-alkylene (for example, methylene), wherein the hydrogen atom in $X^f$ is optionally substituted by one or more substituents selected from F, $C_{1-3}$-alkyl and $C_{1-3}$-fluoroalkyl, and preferably substituted;
$X^{10}$ each independently is a single bond, -O-$R^{36}$-O-, - $R^{36}$-, or o-, m-, or p-phenylene grouppreferably a single bond, -O-$R^{36}$-O-, or -$R^{36}$-, more preferably a single bond or -$R^{36}$-, and still more preferably a single bond;
$R^{36}$ is $C_{1-20}$-alkylene, preferably $C_{1-3}$-alkylene, and still more preferably $C_{1-2}$-alkylene, each optionally substituted by one or more F, wherein the alkylene group in $R^{36}$ may be linear or branched;
$X^{11}$ each independently is a single bond, O, or $C_{1-20}$-alkylene optionally substituted by one or more F, preferably a single bond or unsubstituted $C_{1-6}$-alkylene, more preferably a single bond or unsubstituted $C_{1-3}$-alkylene, and particularly preferably unsubstituted $C_{1-3}$-alkylene;
$R^{34}$ each independently is H, phenyl or $C_{1-6}$-alkyl, preferably H or $C_{1-6}$-alkyl, more preferably H or methyl, and still more preferably H;
$R^{35}$ each independently is $C_{1-6}$-alkylene, and preferably $C_{1-3}$-alkylene;
n4 each independently is an integer of 1-5, preferably 1-3, and more preferably 1;
Y each independently is -$(CH_2)_{g'}$-O-$(CH_2)_{h'}$- (wherein g' is an integer of 0-6, and h' is an integer of 0-6 (e.g. 1-6) or $C_{1-6}$-alkylene (preferably $C_{2-3}$-alkylene) ;
12 is 3; and
n2 is 3.

In the embodiment, g' may be 0.

In one embodiment,

PFPE each independently is -$(OCF(CF_3)CF_2)_d$-
(wherein d is an integer of 1-200, preferably 5-200, and more preferably 10-200;
$X^5$ is a single bond;
Y each independently is -$(CH_2)_{g'}$-O-$(CH_2)_{h'}$- (wherein g' is an integer of 0-6, for example, g' is 0, and h' is an integer of 0-6 (e.g. 1-6) or $C_{1-6}$-alkylene (preferably $C_{2-3}$-alkylene);
12 is 3; and
n2 is 3.

In a preferred embodiment, in the formulae (C1) and (C2),

PFPE each independently is -$(OCF(CF_3)CF_2)_d$- wherein d is an integer of 1-200, preferably 5-200, and more preferably 10-200;

$X^5$ each independently is $-X^f-X^{10}-CON(R^{34})-X^{11}-$, $-X^f-X^{10}-(OR^{35})_{n4}-X^{11}-$, or $-X^f-(C_{1-6}$-alkylene, preferably propylene), and preferably $-X^f-X^{10}-CON(R^{34})-X^{11}-$;

$X^f$ each independently is a single bond or $C_{1-6}$-alkylene, preferably a single bond or $C_{1-4}$-alkylene, and more preferably a single bond or $C_{1-2}$-alkylene (for example, methylene), wherein the hydrogen atom in $X^f$ is optionally substituted by one or more substituents selected from a F, $C_{1-3}$-alkyl and $C_{1-3}$-fluoroalkyl, and preferably substituted;

$X^{10}$ each independently is a single bond, $-O-R^{36}-O-$, $-R^{36}-$, or o-, m-, or p-phenylene, preferably a single bond, $-O-R^{36}-O-$, or $-R^{36}-$, more preferably a single bond or $-R^{36}-$, and still more preferably a single bond;

$R^{36}$ is $C_{1-20}$-alkylene, preferably $C_{1-3}$-alkylene, and still more preferably $C_{1-2}$-alkylene, each optionally substituted by one or more F, wherein the alkylene group in $R^{36}$ may be linear or branched;

$X^{11}$ each independently is a single bond, O, or $C_{1-20}$-alkylene optionally substituted by one or more F, preferably a single bond or unsubstituted $C_{1-6}$-alkylene, more preferably a single bond or unsubstituted $C_{1-3}$-alkylene, and particularly preferably unsubstituted $C_{1-3}$-alkylene;

$R^{34}$ each independently is H, phenyl or $C_{1-6}$-alkyl, preferably H or $C_{1-6}$-alkyl, more preferably H or methyl, and still more preferably H;

$R^{35}$ each independently is $C_{1-6}$-alkylene, and preferably $C_{1-3}$-alkylene;

n4 each independently is an integer of 1-5, preferably 1-3, and more preferably 1; and

Y is $C_{1-6}$-alkylene, and preferably $C_{2-3}$-alkylene.

In a preferred embodiment, the compounds (C1) and (C2) are compounds (C1).

In one embodiment, the PFPE-containing silane compound of formula (C1) or (C2) may be produced by combining known methods.

In one embodiment, the PFPE-containing silane compound of the present disclosure is a compound of formula (B1), (B2), (C1) or (C2).

In the embodiment, the formula (B1) is preferably represented by the following formula (B1").

$$Rf-PFPE-X^3-SiR^a_3 \qquad (B1")$$

In the formula (B1"):

Rf is independently at each occurrence $CF_2H-(C_{1-15}$-fluoroalkylene) or $C_{1-16}$-perfluoroalkyl, and preferably $C_{1-16}$-perfluoroalkyl;

$X^3$ is $-X^f-X^{10}-CON(R^{34})-X^{11}-$, $-X^f-X^{10}-(OR^{35})_{n4}-X^1-$, or $-X^f-(C_{1-6}$-alkylene, preferably propylene), and preferably $-X^f-X^{10}-CON(R^{34})-X^{11}-$;

$X^f$ each independently is a single bond or $C_{1-6}$-alkylene, preferably a single bond or $C_{1-4}$-alkylene, and more preferably a single bond or $C_{1-2}$-alkylene (for example, methylene), wherein the hydrogen atom in $X^f$ is optionally substituted by one or more substituents selected from a F, $C_{1-3}$-alkyl and $C_{1-3}$-fluoroalkyl, and preferably substituted;

$X^{10}$ each independently is a single bond, $-O-R^{36}-O-$, $-R^{36}-$, or o-, m-, or p-phenylene, preferably a single bond, $-O-R^{36}-O-$, or $-R^{36}-$, more preferably a single bond or $-R^{36}-$, and still more preferably a single bond;

$R^{36}$ is $C_{1-20}$-alkylene, preferably $C_{1-3}$-alkylene, and still more preferably $C_{1-2}$-alkylene, each optionally substituted by one or more F, wherein the alkylene group in $R^{36}$ may be linear or branched;

$X^{11}$ each independently is a single bond, O, or a $C_{1-20}$-alkylene optionally substituted by one or more F, preferably a single bond or unsubstituted $C_{1-6}$-alkylene, more preferably a single bond or unsubstituted $C_{1-3}$-alkylene, and particularly preferably unsubstituted $C_{1-3}$-alkylene;

$R^{34}$ each independently is H, phenyl or $C_{1-6}$-alkyl, preferably H or $C_{1-6}$-alkyl, more preferably H or methyl, and still more preferably H;

$R^{35}$ each independently is $C_{1-6}$-alkylene, and preferably $C_{1-3}$-alkylene;

n4 each independently is an integer of 1-5, preferably 1-3, and more preferably 1; and

$R^a$ each independently at each occurrence represents $-Z^3-SiR^{72}_3$;

$Z^3$ is preferably a $C_{1-6}$-alkylene or -phenylene-$(CH_2)_i$-, more preferably $C_{1-6}$-alkylene, and still more preferably $C_{1-3}$-alkylene;

$R^{72}$ is a hydrolyzable group, and more preferably -OR (wherein R is optionally substituted $C_{1-3}$-alkyl, and more preferably methyl);

In the embodiment, $X^3$ may be $-X^f-X^{10}-(OR^{35})_{n4}-X^{11}-$. In the formula, $X^f$, $X^{10}$, $X^{11}$, $R^{35}$, and n4 are as defined above.

In the embodiment, the formula (B2) is preferably of the following formula (B2").

$$R^a_3Si-X^3-PFPE-X^3-SiR^a_3 \qquad (B2")$$

In the formula (B2"), each symbol is as defined in the formula (B1").

In the embodiment, the formula (C1) is preferably represented by the following formula (C1").

$$Rf\text{-}PFPE\text{-}X^5\text{-}CR^d_{k2}R^e_3 \qquad (C1")$$

In the formula (C1");

Rf is independently at each occurrence $CF_2H$-($C_{1-15}$-fluoroalkylene) or $C_{1-16}$-perfluoroalkyl, and preferably $C_{1-16}$-perfluoroalkyl; $X^5$ is -$X^f$-$X^{10}$-CON($R^{34}$) -$X^{11}$-, -$X^f$-$X^{10}$-($OR^{35}$)$_{n4}$-$X^{11}$-, or -$X^f$-($C_{1-6}$-alkylene, preferably propylene), and preferably -$X^f$-$X^{10}$-CON ($R^{34}$) -$X^{11}$-;

$X^f$ each independently is a single bond or $C_{1-6}$-alkylene, preferably a single bond or $C_{1-4}$-alkylene, and more preferably a single bond or $C_{1-2}$-alkylene (for example, methylene), wherein the hydrogen atom in $X^f$ is optionally substituted by one or more substituents selected from a F, $C_{1-3}$-alkyl and $C_{1-3}$-fluoroalkyl, and preferably substituted;

$X^{10}$ is each independently at each occurrence a single bond, -O-$R^{36}$-O-, -$R^{36}$-, or o-, m-, or p-phenylene, preferably a single bond, -O-$R^{36}$-O-, or -$R^{36}$-, more preferably a single bond or -$R^{36}$-, and still more preferably a single bond;

$R^{36}$ is $C_{1-20}$-alkylene, preferably $C_{1-3}$-alkylene, and still more preferably $C_{1-2}$-alkylene, each optionally substituted by one or more F, wherein the alkylene group in $R^{36}$ may be linear or branched;

$X^{11}$ each independently is a single bond, O, or a $C_{1-20}$-alkylene optionally substituted by one or more F, preferably a single bond or unsubstituted $C_{1-6}$-alkylene, more preferably a single bond or unsubstituted $C_{1-3}$-alkylene, and particularly preferably unsubstituted $C_{1-3}$-alkylene;

$R^{34}$ each independently is H, phenyl or $C_{1-6}$-alkyl, preferably H or $C_{1-6}$-alkyl, more preferably H or methyl, and still more preferably H;

$R^{35}$ each independently is $C_{1-6}$-alkylene, and preferably $C_{1-3}$-alkylene;

n4 each independently is an integer of 1-5, preferably 1-3, and more preferably 1; and

$R^e$ is -Y-$SiR^{85}_3$;

Y is $C_{1-6}$-alkylene, and preferably $C_{2-3}$-alkylene;

$R^{85}$ each independently is OH or a hydrolyzable group, and preferably -OR (wherein R is optionally substituted $C_{1-3}$-alkyl, more preferably methyl or ethyl, and particularly methyl).

In the embodiment, formula (C2) is preferably of formula (C2").

$$R^e_3C\text{-}X^5\text{-}PFPE\text{-}X^5\text{-}CR^e_3 \qquad (C2")$$

In the formula (C2"), each symbol is as defined in the formula (C1").

In one embodiment, the present silane compound is a compound of formula (C1) or (C2).

In the embodiment, preferably, the compound (C1) is a compound of formula (C1") and the compound (C2) is a compound of formula (C2").

In a preferred embodiment, the present silane compound is a compound of the formula (B1) or (C1).

More preferably, the present silane compound is a compound of formula (C1).

In the embodiment, preferably, the compound of formula (C1) is a compound of formula (C1").

Methods suitable for producing the present silane compounds are described below by way of example, but the methods of the present disclosure are not limited to the following.

In one embodiment, a method suitable for producing the PFPE-containing silane compound of formula (C1) or (C2) and including the following steps will be described.

Step (3):

a step of reacting a compound of the formula (c1-3) or (c2-3):

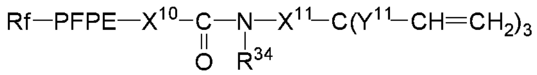

(c1-3)

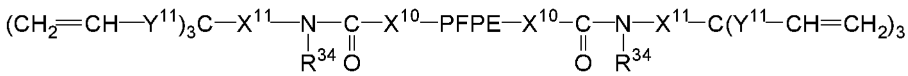

(c2-3)

wherein, each independently at each occurrence,

Rf is $C_{1-16}$-alkyl optionally substituted by F;
PFPE is $(-OCF(CF_3)CF_2-)_d$ wherein d is an integer of 2-200;
$X^{10}$ is a single bond or a divalent organic group;
$R^{34}$ is H, phenyl or $C_{1-6}$-alkyl;
$X^{11}$ is a single bond or a divalent organic group; and
$Y^{11}$ is a single bond or a divalent organic group; with $HSiM_3$, wherein M' each independently is halogen

or $C_{1-6}$-alkoxy,
and, optionally,
a compound of the formula: $R^{85}{}_iL'$ wherein $R^{85}$ each independently is OH or a hydrolyzable group; L' is a group capable of binding to $R^{85}$; and i is an integer of 1-3,
and/or
a compound of the formula: $R^{86'}{}_jL''$ wherein $R^{86'}$ is $C_{1-22}$-alkyl; L" is a group capable of binding to $R^{86'}$; and j is an integer of 1-3.

It is noted that $-X^{10}-C(O)N(R^{34})-X^{11}-$ corresponds to $X^5$ in the formulae (C1) and (C2) and $-Y^{11}-CH_2CH_2-$ corresponds to Y in the formulae (C1) and (C2).
In the embodiment, preferably, the compound of formula (c1-3) or (c2-3) is produced by a method including the following steps (1) and (2).

Step (1):
reacting a compound of formula (c1-1) or (c2-1):

$$Rf\text{-}PFPE\text{-}X^{10}\text{-}C(O)OH \qquad (c1\text{-}1)$$

$$HOC(O)\text{-}X^{10}\text{-}PFPE\text{-}X^{10}\text{-}C(O)OH \qquad (c2\text{-}1)$$

wherein:

Rf, PFPE, and $X^{10}$ are as defined above,
with $SOM_2$,
wherein M each independently is Cl or F,
to obtain a compound of formula (c1-2) or (c2-2):

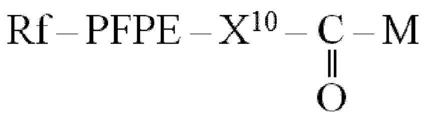

···(c1-2)

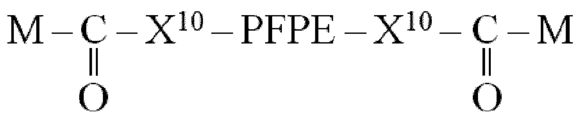

···(c2-2)

wherein:
Rf, PFPE, $X^{10}$, and M are as defined above; and
Step (2):
reacting the compound of formula (c1-2) or (c2-2) with a compound of the formula:

$$HN(R^{34})\text{-}X^{11}\text{-}C(Y^{11}\text{-}CH{=}CH_2)_3,$$

wherein $R^{34}$, $X^{11}$, and $Y^{11}$ are as defined above,
to obtain a compound of formula (c1-3) or (c2-3):

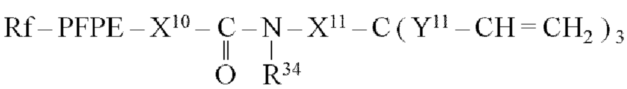

···(c1-3)

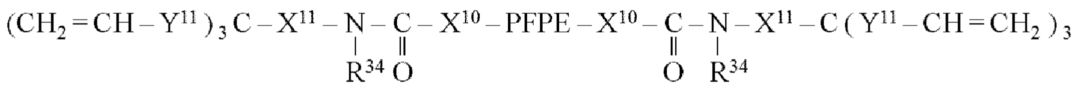

$\cdots$(c2-3)

wherein Rf, PFPE, $X^{10}$, $R^{34}$, and $X^{11}$ are as defined above.

The above producing method will be described in detail below.

Step (1):

In formula (c1-1) or (c2-1), Rf is as defined above.
In formula (c1-1) or (c2-1), PFPE is as defined above.
In formula (c1-1) or (c2-1), $X^{10}$ each independently is at each occurrence a single bond or a divalent organic group, preferably a single bond or $C_{1-6}$-alkylene optionally substituted by one or more F, and more preferably $C_{1-3}$-alkylene which is optionally substituted by one or more F.
The compound of formula (c1-1) or (c2-1) is commercially available, or may be produced from a commercially available compound using conventional techniques in the art.
In $SOM_2$ used in step (1), M is each independently at each occurrence Cl or F, and preferably Cl. The compound is commercially available, or may be produced from a commercially available compound using conventional techniques in the art.
The amount of $SOM_2$ used in step (1) is preferably ≥ 1 mol per mol of the terminal COOH group (as the total amount when two or more compounds are used; the same applies below) of the compound of formula (c1-1) and/or (c2-1).
The reaction of step (1) is preferably carried out in the presence of a suitable catalyst in a suitable solvent.
Examples of the suitable catalyst include N,N-dimethylformamide (DMF).
The suitable solvent is not limited as long as it does not adversely affect the reaction, and examples thereof include 1,3-bis(trifluoromethyl)benzene, perfluorobutyl ethyl ether, and perfluorohexyl methyl ether.
The reaction temperature in such a reaction is usually, but not limited to, 0-150°C, preferably 80-100°C, and the reaction time is usually, but not limited to, 30-600 minutes, preferably 60-120 minutes, and the reaction pressure is, but not limited to, -0.2 to 1 MPa (gauge pressure), and is conveniently atmospheric pressure.

Step (2):

In the compound $HN(R^{34})$-$X^{41}$-$C(Y^{11}$-$CH=CH_2)_3$ used in step (2), $X^{11}$ is a single bond or a divalent organic group, preferably a single bond or $C_{1-6}$-alkylene optionally substituted by one or more F, and more preferably $C_{1-3}$-alkylene group.
In the formula, $R^{34}$ each independently is H, phenyl, or $C_{1-6}$-alkyl, preferably H or $C_{1-6}$-alkyl group, more preferably H or methyl, and still more preferably H.
In the formula, $Y^{11}$ each independently is a single bond or a divalent organic group. $Y^{11}$ is preferably $C_{1-6}$-alkylene, and more preferably $C_{1-3}$-alkylene.
Specific examples of the compound: $HN(R^{34})$-$X^{11}$-$C(Y^{11}$-$CH=CH_2)_3$ used in step (2) include $H_2NCH_2C(CH_2$-$CH=CH_2)_3$, $H_2NCH_2CH_2C(CH_2$-$CH=CH_2)_3$, and $H_2NCH_2CH_2CH_2C(CH_2$-$CH=CH_2)_3$.
The amount of the compound of the formula $HN(R^{34})$-$X^{11}$-$C(Y^{11}$-$CH=CH_2)_3$ is preferably ≥ 1 mol per mol of the terminal - C(O)M group of the compound of formula (c1-2) and/or (c2-2).
The reaction of step (2) is preferably carried out in the presence of a suitable catalyst in a suitable solvent.
Examples of suitable catalyst include triethylamine and diisopropylethylamine.
The suitable solvent is not limited as long as it does not adversely affect the reaction, and examples thereof include 1,3-bis(trifluoromethyl)benzene, perfluorobutyl ethyl ether, and perfluorohexyl methyl ether.
The reaction temperature in such a reaction is usually, but not limited to, 0-100°C, preferably 0-40°C, and the reaction time is usually, but not limited to, 30-600 minutes, preferably 60-120 minutes, and the reaction pressure is, but not limited to, -0.2 to 1 MPa (gauge pressure), and is conveniently atmospheric pressure.

Step (3):

In step (3), the compound of formula (c1-3) or (c2-3) is reacted with $HSiM'_3$ and, optionally, a compound of the formula $R^{85}{}_iL'$, and/or a compound of the formula: $R^{86'}{}_jL''$.
The compound of formula (c1-3) or (c2-3) is preferably the compound obtained in the step (2).

In the compound $HSiM'_3$ used in step (3), M' each independently is halogen or $C_{1-6}$-alkoxy, preferably halogen, and more preferably Cl.

In the compound of the formula $R^{85}{}_iL'$ used in step (3), $R^{85}$ is as described in the formulae (C1) and (C2); L' is a group capable of binding to $R^{85}$; and i is an integer of 1-3.

In the compound of the formula $R^{86'}{}_jL''$ used in step (3), $R^{86'}$ is $C_{1-22}$-alkyl; L" is a group capable of binding to $R^{86'}$; and j is an integer of 1-3.

The "group capable of binding to $R^{85}$" and the "group capable of binding to $R^{86'}$" represented by L' and L" above are capable of binding to $R^{85}$ and $R^{86'}$, respectively, and $R^{85}$ and $R^{86'}$ are not limited as long as $R^{85}$ and $R^{86'}$ can leave from these groups in the above reaction, and examples thereof include a hydrogen atom, lithium, and sodium. The "group capable of binding to $R^{85}$" and the "group capable of binding to $R^{86'}$" may be a group which may have a plurality of $R^{85}$ or $R^{86'}$ groups, for example, $=CH_2$, $\equiv CH$. Those skilled in the art can select an appropriate group capable of binding to $R^{85}$ and a group capable of binding to $R^{86'}$ depending on the conditions such as the type of compound to be reacted, the solvent, and the temperature.

The amount of $HSiM'_3$ used in step (3) may be ≥ 1 mol, but is preferably 2 mol, per mol of the terminal $-CH=CH_2$ group of the compound of formula (c1-3) and/or (c2-3).

When the compound $R^{85}{}_iL'$ is used in step (3), the use amount thereof may vary depending on the amount of $R^{85}$ group to be introduced, and such amount can be appropriately determined by those skilled in the art.

When the compound $R^{86'}{}_jL''$ is used in step (3), the use amount thereof may vary depending on the amount of $R^{86'}$ group to be introduced, and such amount can be appropriately determined by those skilled in the art.

In the reaction of step (3), firstly, the terminal - $CH=CH_2$ group of the compound of formula (c1-3) and/or (c2-3) is reacted with $HSiM'_3$ to convert its end to the $-CH_2CH_2SiM'_3$ group. Then, this terminal $-CH_2CH_2SiM'_3$ group is reacted with the compound $R^{85}{}_iL'$ and/or the compound $R^{86'}{}_jL''$ to replace M' with $R^{85}$ or $R^{86'}$. It is noted that the compound $R^{85}{}_iL'$ and the compound $R^{86'}{}_jL''$ may be reacted simultaneously or separately.

However, in one embodiment of the present disclosure, $HSiM'_3$, the compound represented by $R^{85}{}_iL'$, and the compound $R^{86'}{}_jL''$ may be used as a compound of $HSi(R^{85}{}_i)(R^{86'}{}_j)$ (in this case, i+j is 3). The compound $HSi(R^{85}{}_i)(R^{86'}{}_j)$ may be produced by those skilled in the art using conventional techniques in the art.

In another embodiment, the total amount of the compound represented by $R^{85}{}_iL'$ and/or the compound represented by $R^{86'}{}_jL''$ in step (3) is set to ≥ 3 mol per mol of the terminal $-CH=CH_2$ group of the compound of formula (c1-3) and/ or (c2-3). According to such an embodiment, substantially all M' of the terminal $-CH_2CH_2SiM'_3$ produced in the reaction of the step (3) can be replaced with $R^{85}$ or $R^{86'}$.

The reaction in step (3) is preferably carried out in the presence of a suitable catalyst in a suitable solvent.

Examples of suitable catalyst include Pt, Pd, and Rh. The catalyst is preferably Pt. Such catalyst may be in any form, for example in the form of a complex.

The suitable solvent is not limited as long as it does not adversely affect the reaction, and examples thereof include 1,3-bis(trifluoromethyl)benzene, perfluorobutyl ethyl ether, and perfluorohexyl methyl ether.

The reaction temperature in such a reaction is usually, but not limited to, 0-100°C, and preferably 50-80°C, and the reaction time is usually, but not limited to, 30-600 minutes, and preferably 60-240 minutes, and the reaction pressure is, but not limited to, -0.2 to 1 MPa (gauge pressure), and is conveniently atmospheric pressure.

The reaction of step (3) is preferably carried out in the presence of a suitable rearrangement preventing agent.

Examples of the suitable rearrangement preventing agent include carboxylic acid compounds. The carboxylic acid compound may include (a) a carboxylic acid, (b) a carboxylic acid anhydride, (c) a silylated carboxylic acid, and/or (d) a substance that produces the carboxylic acid compounds (i.e., (a), (b) or (c)) in the reaction of step (3). These carboxylic acid compounds may be used alone or in combination of two or more.

When the rearrangement preventing agent contains (a) the carboxylic acid, any carboxylic acid having a carboxyl group may be used. Examples of the suitable carboxylic acid include saturated carboxylic acids, unsaturated carboxylic acids, monocarboxylic acids, and dicarboxylic acids. Specific examples of the suitable carboxylic acid include a saturated monocarboxylic acid such as formic acid, acetic acid, propionic acid, n-butyric acid, isobutyric acid, hexanoic acid, cyclohexanoic acid, lauric acid, and stearic acid; a saturated dicarboxylic acid such as oxalic acid and adipic acid; an aromatic carboxylic acid such as benzoic acid and p-phthalic acid; a carboxylic acid in which the hydrogen atom on the carbon of the hydrocarbon group of these carboxylic acids has been substituted by a halogen atom or an organosilyl group, such as chloroacetic acid, dichloroacetic acid, trifluoroacetic acid, para-chlorobenzoic acid, and trimethylsilylacetic acid; an unsaturated fatty acid such as acrylic acid, methacrylic acid, and oleic acid; and compounds having a hydroxy group, a carbonyl group or an amino group in addition to the carboxyl group, namely, a hydroxy acid such as lactic acid, a keto acid such as acetoacetic acid, an aldehyde acid such as glyoxylic acid, and an amino acid such as glutamic acid.

Examples of (b) the carboxylic acid anhydride include acetic acid anhydride, propionic acid anhydride, and benzoic acid anhydride. These carboxylic acid anhydrides may be those generated in the reaction system of step (3), and include acetyl chloride, butyryl chloride, benzoyl chloride, and other carboxylic acid halides; carboxylic acid metal salts such as zinc acetate and thallium acetate; and carboxylic esters decomposable by light or heat, such as (2-nitrobenzyl)propionate.

Examples of (c) the silylated carboxylic acid include trialkylsilylated carboxylic acids, such as trimethylsilyl formate, trimethylsilyl acetate, triethylsilyl propionate, trimethylsilyl benzoate, and trimethylsilyl trifluoroacetate; and di-, tri-, or tetracarboxysilylates, such as dimethyldiacetoxysilane, diphenyldiacetoxysilane, methyltriacetoxysilane, ethyltriacetoxysilane, vinyltriacetoxysilane, di-t-butoxydiacetoxysilane, and silicon tetrabenzoate.

The rearrangement preventing agent is used in an amount of 0.001-20 wt.%, for example 0.01-5 wt.%, or 0.01-1 wt.%, but not limited thereto. Those skilled in the art can select the amount of using the rearrangement preventing agent depending on the compound to be reacted, an agent, a solvent, and other conditions. The rearrangement preventing agent is commercially available as DOW CORNING (registered trademark) ETS 900 or XIAMETER (registered trademark) OFS-1579 Silane available from Dow Corning Corporation of Midland, MI.

In a preferred embodiment, the PFPE is $-(OCF(CF_3)CF_2)_d-$(wherein d is an integer of 2-200, preferably 5-200, more preferably 10-200), and in the production method, the compound of formula (c1-3) is reacted in step (3). More preferably, in the production method, in step (1), the compound of formula (c1-2) is produced from the compound of formula (c1-1); in step (2), the compound of formula (c1-3) is produced from the compound formula (c1-2), and in step (3), the compound of formula (c1-3) is reacted. The operation in each step is as described above.

In one embodiment, the present disclosure provides a method for producing a fluoro(poly)ether group-containing silane compound, which includes the following steps (1)-(3).

Step (1):

a step of reacting a compound of formula (c1-1) or (c2-1) :

$$Rf\text{-}PFPE\text{-}X^{10}\text{-}C(O)OH \qquad (c1\text{-}1)$$

$$HOC(O)\text{-}X^{10}\text{-}PFPE\text{-}X^{10}\text{-}C(O)OH \qquad (c2\text{-}1)$$

wherein Rf, PFPE, and $X^{10}$ are as defined above,
with $SOM_2$,
wherein M is as defined above,
to obtain a compound of formula (c1-2) or (c2-2):

$$Rf-PFPE-X^{10}-\underset{\displaystyle\overset{\|}{O}}{C}-M \qquad \cdots(c1\text{-}2)$$

$$M-\underset{\displaystyle\overset{\|}{O}}{C}-X^{10}-PFPE-X^{10}-\underset{\displaystyle\overset{\|}{O}}{C}-M \qquad \cdots(c2\text{-}2)$$

wherein:
Rf, PFPE, $X^{10}$ and M are as defined above;

Step (2):

a step of reacting the compound of formula (c1-2) or (c2-2) with a compound of the formula:

$$HN(R^{34})\text{-}X^{11}\text{-}C(Y^{11}\text{-}CH{=}CH_2)_3,$$

wherein $R^{34}$, $X^{11}$, and $Y^{11}$ are as defined above,
to obtain a compound of formula (c1-3) or (c2-3):

$$Rf-PFPE-X^{10}-\underset{\displaystyle\overset{\|}{O}}{C}-\underset{\displaystyle\overset{|}{R^{34}}}{N}-X^{11}-C(Y^{11}-CH=CH_2)_3 \qquad \cdots(c1\text{-}3)$$

$$(CH_2=CH-Y^{11})_3C-X^{11}-N(R^{34})-C(=O)-X^{10}-PFPE-X^{10}-C(=O)-N(R^{34})-X^{11}-C(Y^{11}-CH=CH_2)_3 \quad \cdots (c2\text{-}3)$$

wherein Rf, PFPE, $X^{10}$, $R^{34}$, and $X^{11}$ are as defined above; and

Step (3):

a step of reacting the compound of formula (c1-3) or (c2-3):

$$Rf-PFPE-X^{10}-C(=O)-N(R^{34})-X^{11}-C(Y^{11}-CH=CH_2)_3 \quad \cdots (c1\text{-}3)$$

$$(CH_2=CH-Y^{11})_3C-X^{11}-N(R^{34})-C(=O)-X^{10}-PFPE-X^{10}-C(=O)-N(R^{34})-X^{11}-C(Y^{11}-CH=CH_2)_3 \quad \cdots (c2\text{-}3)$$

with $HSiM_3$,
and, optionally,
a compound of the formula $R^{85}{}_iL'$
and/or
a compound of the formula $R^{86'}{}_jL''$
wherein M', $R^{85}$, L', i, $R^{86'}$, L", and j are as defined above.

The present disclosure further provides a compound of formula (c1-2) or (c2-2):

$$Rf-PFPE-X^{10}-C(=O)-M \quad \cdots (c1\text{-}2)$$

$$M-C(=O)-X^{10}-PFPE-X^{10}-C(=O)-M \quad \cdots (c2\text{-}2)$$

that is, an intermediate in the production method.

In the formula, Rf, PFPE, $X^{10}$, and M are as defined above.

Preferably, the intermediate is a compound of formula (c1-2) .

The present disclosure still further provides a compound of formula (c1-3) or (c2-3):

$$Rf-PFPE-X^{10}-C(=O)-N(R^{34})-X^{11}-C(Y^{11}-CH=CH_2)_3 \quad \cdots (c1\text{-}3)$$

$$(CH_2=CH-Y^{11})_3C-X^{11}-N(R^{34})-C(=O)-X^{10}-PFPE-X^{10}-C(=O)-N(R^{34})-X^{11}-C(Y^{11}-CH=CH_2)_3 \quad \cdots (c2\text{-}3)$$

that is, an intermediate in the production method.

In the formula, Rf, PFPE, $X^{10}$, $R^{34}$, $X^{11}$, and $Y^{11}$ are as defined above.

Preferably, the intermediate is a compound offormula (c1-3) .

(surface-treating agent)

The surface-treating agent of the present disclosure contains a PFPE-containing silane compound of formula (A1), (A2), (B1), (B2), (C1), or (C2).

The surface-treating agent can contribute to the formation of a surface-treating layer having good UV durability, water-repellency, oil-repellency, antifouling property (for example, preventing adhesion of fouling such as fingerprints), chemical resistance, hydrolysis resistance, an effect of suppressing lubricity, high friction durability, heat resistance and moisture-proof property.

The surface-treating agent may be diluted with a solvent. Examples of the solvent include:
a fluorine atom-containing solvent selected from perfluorohexane, $CF_3CF_2CHCl_2$, $CF_3CH_2CF_2CH_3$, $CF_3CHFCHFC_2F_5$, 1,1,1,2,2,3,3,4,4,5,5,6,6-tridecafluorooctane, 1,1,2,2,3,3,4-heptafluorocyclopentane (e.g. ZEORORA H (trade name)), $C_4F_9OCH_3$, $C_4F_9OC_2H_5$, $CF_3CH_2OCF_2CHF_2$, $C_6F_{13}CH=CH_2$, $C_6F_{13}OCH_3$, xylene hexafluoride, perfluorobenzene, methyl pentadecafluoroheptyl ketone, trifluoroethanol, pentafluoropropanol, hexafluoroisopropanol, $HCF_2CF_2CH_2OH$, methyl trifluoromethanesulfonate, trifluoroacetic acid, and $CF_3O(CF_2CF_2O)m1(CF_2O)_{n1}CF_2CF_3$ (wherein m1 and n1 each independently are an integer of 0-1000, the order of the units in parentheses with m1 or n1 is arbitrary, with the proviso that (m1 + n1) is ≥ 1), 1,1-dichloro-2,3,3,3-tetrafluoro-l-propene, 1,2-dichloro-1,3,3,3-tetrafluoro-1-propene, 1,2-dichloro-3,3,3-trifluoro-1-propene, 1,1-dichloro-3,3,3-trifluoro-1-propene, 1,1,2-trichloro-3,3,3-trifluoro-1-propene, and 1,1,1,4,4,4-hexafluoro-2-butene. These solvents may be used alone or as a mixture of two or more compounds.

The content of moisture contained in the solvent is preferably ≤ 20 mass-ppm. The content of moisture may be measured using the Karl Fischer method. With such a content of moisture, the storage stability of the surface-treating agent can be improved.

The surface-treating agent may contain other components in addition to the present silane compound of formula (A1), (A2), (B1), (B2), (C1), or (C2). Examples of the other components include a (non-reactive) fluoropolyether compound which may be also understood as fluorine-containing oils, preferably perfluoro(poly)ether compounds (hereinafter, referred to as "the fluorine-containing oil"), (non-reactive) silicone compounds which may be also understood as silicone oils (hereinafter referred to as "silicone oil"), catalysts, lower alcohols, transition metals, halide ions, and compounds containing an atom having an unshared electron pair in a molecular structure.

Examples of the fluorine-containing oil include a a perfluoro(poly)ether compound of formula (1):

$$Rf^5\text{-}(OC_4F_8)_{a'}\text{-}(OC_3F_6)_{b'}\text{-}(OC_2F_4)_{c'}\text{-}(OCF_2)_{d'}\text{-}Rf^6 \qquad (1)$$

wherein $Rf^5$ is $C_{1\text{-}16}$-alkyl optionally substituted by one or more F (preferably, $C_{1\text{-}16}$-perfluoroalkyl), $Rf^6$ is H, F or $C_{1\text{-}16}$-alkyl optionally substituted by one or more F (preferably, $C_{1\text{-}16}$-perfluoroalkyl), and more preferably, $Rf^5$ and $Rf^6$ each independently $C_{1\text{-}3}$-perfluoroalkyl.
a', b', c', and d' represent the repeating number of each of four repeating units of perfluoro(poly)ether which constitute a main backbone of the polymer, and are each independently an integer of 0-300, and (a'+b'+c'+d') is ≥ 1, preferably 1-300, and more preferably 20-300. The order of the units in parentheses with the subscript a', b', c', or d' is arbitrary. There is at least one branched structure in the repeating units. That is, the repeating units have at least one $CF_3$ end (specifically, $-CF_3$ or $-C_2F_5$, more specifically $-CF_3$). Regarding the repeating unit having a branched structure, examples of $-(OC_4F_8)-$ include - $(OCF(CF_3)CF_2CF_2)$, $-(OCF_2CF(CF_3)CF_2)-$, $-(OCF_2CF_2CF(CF_3))-$, - $(OC(CF_3)_2CF_2)-$, $-(OCF_2C(CF_3)_2)-$, $-(OCF(CF_3)CF(CF_3))-$, - $(OCF(C_2F_5)CF_2)-$, and - $(OCF_2CF(C_2F_5))-$; and examples of - $(OC_3F_6)-$ include $-(OCF(CF_3)CF_2)-$ and - $(OCF_2CF(CF_3))-$; and examples of $-(OC_2F_4)-$ include - $(OCF(CF_3))-$.

Examples of the compound of formula (1) include a compound of any of the formulae (1a) and (1b) (may be one compound or a mixture of two or more compounds).

$$Rf^5\text{-}(OCF(CF_3)CF_2)_{b''}\text{-}Rf^6 \qquad (1a)$$

$$Rf^5\text{-}(OC_4F_8)a''\text{-}(OC_3F_6)b''\text{-}(OCF(CF_3))_{c''}\text{-}(OCF_2)_{d''}\text{-}Rf^6 \qquad (1b)$$

In these formulae, $Rf^5$ and $Rf^6$ are as defined above; in the formula (1a), b'' is an integer of 1-100; and in the formula (1b), a'' and b'' each independently are an integer of 1-30, and c'' and d'' each independently are an integer of 1-300. The order of the units in parentheses with the subscript a'', b'', c'', or d'' is arbitrary. $-(OC_4F_8)-$ and - $(OC_3F_6)-$ have a branched structure.

The fluorine-containing oil may have a number average molecular weight of 1,000-30,000. In particular, the number average molecular weight of the compound formula (1a) is preferably 2,000-8,000. By having such a number average molecular weight, good friction durability can be obtained. In one embodiment, the number average molecular

weight of the compound of formula (1b) is preferably 3,000-8,000. In another embodiment, the number average molecular weight of the compound of formula (1b) is 8,000-30,000.

In the surface-treating agent, the fluorine-containing oil may be contained in an amount of, for example, 0-500 parts by mass (pbm), preferably 0-100 pbm, more preferably 1-50 pbm, and still more preferably 1-5 pbm based on 100 pbm of the PFPE-containing silane compound.

From the other point of view, the fluorine-containing oil may be a compound of the formula Rf'-F (wherein Rf' is $C_{5-16}$-perfluoroalkyl). In addition, the fluorine-containing oil may be a chlorotrifluoroethylene oligomer. The compound represented by Rf'-F and the chlorotrifluoroethylene oligomer are preferable in that the compound shows high affinity to the above-mentioned fluoro(poly)ether group-containing silane compound in which Rf is $C_{1-16}$.perfluoroalkyl.

The fluorine-containing oil contributes to improving the surface lubricity of the surface-treating layer.

Examples of the silicone oil include a linear or cyclic silicone oil having $\leq$ 2,000 siloxane bonds. The linear silicone oil may be so-called a straight silicone oil and a modified silicon oil. Examples of the straight silicone oil include dimethylsilicone oil, methylphenylsilicone oil, and methylhydrogensilicone oil. Examples of the modified silicone oil include that which is obtained by modifying a straight silicone oil with alkyl, aralkyl, polyether, higher fatty acid ester, fluoroalkyl, amino, epoxy, carboxyl or alcohol. Examples of cyclic silicone oil include cyclic dimethylsiloxane oil.

The silicone oil may be contained in the surface-treating agent, for example, at 0-50 pbm, and preferably 0-5 pbm based on 100 pbm of the PFPE-containing silane compound (as the total mass when two or more compounds are used; the same applies below).

The silicone oil contributes to improving the surface lubricity of the surface-treating layer.

Examples of the catalyst include an acid (for example, acetic acid and trifluoroacetic acid), a base (for example, ammonia, triethylamine and diethylamine), and a transition metal (for example, Ti, Ni and Sn).

The catalyst facilitates the hydrolysis and dehydration condensation of the fluorine-containing silane compound to facilitate the formation of the surface-treating layer.

Examples of the lower alcohol as the other component include $C_{1-6}$-alcohol compounds.

Examples of the transition metal include platinum, ruthenium, and rhodium.

Examples of the halide ion include chloride ion.

Examples of the compounds containing an atom having an unshared electron pair in the molecular structure include diethylamine, triethylamine, aniline, pyridine, hexamethylphosphoramide, N,N-diethylacetamide, N,N-diethylformamide, N,N-dimethylacetamide, N-methylformamide, N,N-dimethylformamide, N-methylpyrrolidone, tetramethylurea, dimethylsulfoxide (DMSO), tetramethylenesulfoxide, methylphenylsulfoxide, and diphenylsulfoxide. Among these compounds, it is preferable to use dimethyl sulfoxide or tetramethylene sulfoxide.

In addition to the above, examples of the other component include tetraethoxysilane, methyltrimethoxysilane, 3-aminopropyltrimethoxysilane, 3-glycidoxypropyltrimethoxysilane, and methyltriacetoxysilane.

In addition to the above, examples of the other component include a compound (E) having a fluoro(poly)ether group and a carboxylic acid group in the molecular structure. In the compound (E), the fluoro(poly)ether group contains at least one F. By containing such a compound (E), the reaction with glass is facilitated, so that the surface-treating layer can be formed in a shorter time.

Examples of the compound (E) include compounds of formula (E1) or (E2), and specifically, a compound of formula (E1) .

$$Rf^1\text{-}PFPE^1\text{-}X^{f1}\text{-}COOH \qquad (E1)$$

$$HOOC\text{-}X^{f1}\text{-}PFPE^1\text{-}X^{f1}\text{-}COOH \qquad (E2)$$

In the formulae (E1) and (E2),
$Rf^1$ each independently is $C_{1-16}$-alkyl optionally substituted by one or more F. Therein, the " $C_{1-16}$-alkyl " may be linear or branched, and preferably is linear or branched $C_{1-16}$-alkyl, in particular $C_{1-13}$-alkyl, and more preferably linear $C_{1-3}$-alkyl.

$Rf^1$ is preferably $C_{1-16}$-alkyl substituted by one or more F, more preferably $CF_2H$-($C_{1-15}$-fluoroalkylene) or $C_{1-16}$-perfluoroalkyl, and still more preferably $C_{1-16}$-perfluoroalkyl.

The $C_{1-16}$-perfluoroalkyl may be linear or branched, and preferably is linear or branched $C_{1-6}$-perfluoroalkyl, in particular $C_{1-3}$-perfluoroalkyl, and more preferably linear $C_{1-3}$-perfluoroalkyl, specifically $-CF_3$, $-CF_2CF_3$, or $-CF_2CF_2CF_3$.

In the formulae (E1) and (E2),
$PFPE^1$ independently is :
$-(OC_6F_{12})_{a1}-(OC_5F_{10})_{b1}-(OC_4F_8)_{c1}-(OC_3X'_6)_{d1}-(OC_2F_4)_{e1}-(OCF_2)_{f1}-$and has at least one branched structure in $PFPE^1$. That is, the $PFPE^1$ has at least one $CF_3$ end (specifically, $-CF_3$ or $-C_2F_5$, more specifically $-CF_3$). In formula (E1), in the $PFPE^1$, the oxygen atom at the left end of the formula is bonded to the $Rf^1$ group, and the carbon atom at the right end of the formula is bonded to the $X^{f1}$ group.

$X^F$ each independently is H, F or Cl, preferably H or F, and more preferably F.

In the formula, a1, b1, c1, d1, e1, and f1 each independently are an integer of 0-200, and (a1+b1+c1+d1+e1+f1) is ≥ 1. Preferably, a1, b1, c1, d1, e1, and f1 are each independently an integer of 0-100. Preferably, (a1+b1+c1+d1+e1+f1) is ≥ 5, and more preferably ≥ 10. Preferably, (a1+b1+c1+d1+e1+f1) is ≤ 200, more preferably ≤ 100, for example, 10-200, and more specifically 10-100. The order of the units in parentheses with a1, b1, c1, d1, e1, or f1 is arbitrary.

$PFPE^1$ preferably has at least 5, more preferably 10, and particularly preferably 20 branched structures.

In the structure of $PFPE^1$, the number of repeating units having a branched structure is preferably ≥ 40, more preferably ≥ 60, and particularly preferably ≥ 80, with respect to the total number of repeating units (for example, the sum of a1, b1, c1, d1, e1, and f1) of 100. In the structure of $PFPE^1$, the number of repeating units having a branched structure may be ≤ 100, for example, ≤ 90, with respect to the total number of repeating units of 100.

In the structure of $PFPE^1$, the number of repeating units having the branched structure is preferably 40-100, more preferably 60-100, and particularly preferably 80-100, with respect to the total number of repeating units of 100.

Examples of the branched chain in the branched structure include $CF_3$.

Regarding the repeating unit having a branched structure, examples of $-(OC_6F_{12})-$ include $-(OCF(CF_3)CF_2CF_2CF_2CF_2)-$, $-(OCF_2CF(CF_3)CF_2CF_2CF_2)-$, $-(OCF_2CF_2CF(CF_3)CF_2CF_2)-$, $-(OCF_2CF_2CF_2CF(CF_3)CF_2)-$, and $-(OCF_2CF_2CF_2CF_2CF(CF_3))-$. Examples of $-(OC_5F_{10})-$ include $-(OCF(CF_3)CF_2CF_2CF_2)-$, $-(OCF_2CF(CF_3)CF_2CF_2)-$, $-(OCF_2CF_2CF(CF_3)CF_2)-$, and $-(OCF_2CF_2CF_2CF(CF_3))-$. Examples of $-(OC_4F_8)-$ include $-(OCF(CF_3)CF_2CF_2)-$, $-(OCF_2CF(CF_3)CF_2)-$, $-(OCF_2CF_2CF(CF_3))-$, $-(OC(CF_3)_2CF_2)-$, $-(OCF_2C(CF_3)_2)-$, $-(OCF(CF_3)CF(CF_3))-$, $-(OCF(C_2F_5)CF_2)-$, and $-(OCF_2CF(C_2F_5))-$. Examples of $-(OC_3F_6)-$ (i.e., in the formula, $X^F$ is a fluorine atom) include $-(OCF(CF_3)CF_2)-$ and $-(OCF_2CF(CF_3))-$. Examples of $-(OC_2F_4)-$ include $-(OCF(CF_3))-$.

The $PFPE^1$ may include a linear repeating unit as well as a repeating unit having a branched structure. Examples of the linear repeating unit include $-(OCF_2CF_2CF_2CF_2CF_2CF_2)-$, $-(OCF_2CF_2CF_2CF_2CF_2)-$, $-(OCF_2CF_2CF_2CF_2)-$, $-(OCF_2CF_2CF_2)-$, and $-(OCF_2CF_2)-$.

Preferably, in the $PFPE^1$, the repeating units $-(OC_6F_{12})-$, $-(OC_5F_{10})-$, $-(OC_4F_8)-$, and $-(OC_3F_6)-$ have a branched structure.

More preferably, the $PFPE^1$ consists of repeating units $OC_6F_{12}$, $OC_5F_{10}$, $OC_4F_8$, and $OC_3F_6$ having a branched structure.

In one embodiment, the $PFPE^1$ is $-(OC_3F_6)_{d1}-$ (wherein d1 is an integer of 1-200, preferably 5-200, more preferably 10-200), and has at least one branched structure in $PFPE^1$.

In this embodiment, $PFPE^1$ may further contain a linear repeating unit $-(OCF_2CF_2CF_2)-$.

In the embodiment, the $PFPE^1$ preferably consists of a repeating unit $OC_3F_6$ having a branched structure. The $PFPE^1$ is more preferably $-(OCF_2CF(CF_3))_{d1}$, wherein d1 is an integer of 1-200, preferably 5-200, and more preferably 10-200.

In another embodiment, $PFPE^1$ is $-(OC_4F_8)_{c1}-(OC_3F_6)_{d1}-(OC_2F_4)_{e1}-(OCF_2)_{f1}-$ (wherein c1 and d1 each independently are an integer of 0-30, e and f each independently are an integer of 1-200, preferably 5-200, more preferably 10-200, (a1+b1+c1+d1+e1+f1) is ≥ 5, preferably ≥ 10, and the order of the units in parentheses with the subscript c1, d1, e1, or f1 is arbitrary) and has at least one branched structure in $PFPE^1$.

In yet another embodiment, $PFPE^1$ is $-(R^{61}-R^{62})_{j1}-$ and has at least one branched structure in PFPE. In the formula, $R^{61}$ is $OCF_2$ or $OC_2F_4$, and preferably $OC_2F_4$. In the formula, $R^{62}$ is a group selected from $OC_2F_4$, $OC_3F_6$, $OC_4F_8$, $OC_5F_{10}$, and $OC_6F_{12}$, or is a combination of two or three groups independently selected from these groups. Preferably, $R^{62}$ is a group selected from $OC_2F_4$, $OC_3F_6$, and $OC_4F_8$, or is a group selected from $OC_3F_6$, $OC_4F_8$, $OC_5F_{10}$, and $OC_6F_{12}$, or is a combination of two or three groups independently selected from these groups, and more preferably a group selected from $OC_3F_6$ and $OC_4F_8$. Examples of the combination of two or three groups independently selected from $OC_2F_4$, $OC_3F_6$, and $OC_4F_8$ include $-OC_2F_4OC_3F_6-$, $-OC_2F_4OC_4F_8-$, $-OC_3F_6OC_2F_4-$, $-OC_3F_6OC_3F_6-$, $-OC_3F_6OC_4F_8-$, $-OC_4F_8OC_4F_8-$, $-OC_4F_8OC_3F_6-$, $-OC_4F_8OC_2F_4-$, $-OC_2F_4OC_2F_4OC_3F_6-$, $-OC_2F_4OC_2F_4OC_4F_8-$, $-OC_2F_4OC_3F_6OC_2F_4-$, $-OC_2F_4OC_3F_6OC_3F_6-$, $-OC_2F_4OC_4F_8OC_2F_4-$, $-OC_3F_6OC_2F_4OC_2F_4-$, $-OC_3F_6OC_2F_4OC_3F_6-$, $-OC_3F_6OC_3F_6OC_2F_4-$, and $-OC_4F_8OC_2F_4OC_2F_4-$. j1 is ≥ 2, preferably ≥ 3, more preferably ≥ 5, and is an integer of ≤ 100, and preferably ≤ 50. In the formula, $OC_2F_4$, $OC_3F_6$, $OC_4F_8$, $OC_5F_{10}$, and $OC_6F_{12}$ preferably have a branched structure.

More preferably, in the embodiment, $PFPE^1$ consists of repeating units $OC_6F_{12}$, $OC_5F_{10}$, $OC_4F_8$, and $OC_3F_6$ having a branched structure.

In the formulae (E1) and (E2),

$X^{f1}$ each independently is a single bond or $C_{1-6}$-alkylene, preferably a single bond or $C_{1-4}$-alkylene, and more preferably a single bond or $C_{1-2}$-alkylene (for example, methylene). The hydrogen atoms in $X^{f1}$ are optionally substituted, preferably substituted, by one or more substituents selected from F, $C_{1-3}$-alkyl and $C_{1-3}$-fluoroalkyl. $X^{f1}$ may be linear or branched, and is preferably linear.

It is preferable that $Rf^1$ and $PFPE^1$ each have the same structure as Rf or PFPE in the PFPE-containing silane compound contained in the surface-treating agent.

The compound (E) is preferably a compound of formula (E1) .

In the surface-treating agent, the ratio (i):(ii) of (i) the present silane compound to (ii) the compound of formula (E) may be, for example, (99.9:0.1)-(85.0:15.0), (99.9:0.1)-(90.0:10.0), or (99.9:0.1)-(95.0:5.0) in molar ratio.

In one embodiment, the surface-treating agent does not contain the other components: fluorine-containing oils, silicone oils, catalysts, lower alcohols, transition metals, halide ions, and compounds containing an atom having an unshared electron pair in the molecular structure.

In one embodiment, the surface-treating agent contains a PFPE-containing silane compound of formula (B1), (B2), (C1) or (C2).

In one embodiment, the surface-treating agent contains a PFPE-containing silane compound of formula (C1) or (C2).

In one embodiment, the surface-treating agent contains a PFPE-containing silane compound of formula (C1).

In one embodiment, the surface-treating agent contains a PFPE-containing silane compound of formula (B1) or (C1), and preferably a PFPE-containing silane compound of formula (C1) .

In one embodiment, the surface-treating agent contains a PFPE-containing silane compound of formula (C1) and does not contain fluorine-containing oil (for example, the content of the fluorine-containing oil is ≤ 1 pbm, more specifically 0 pbm, based on 100 pbm of the surface-treating agent) which is the other component.

The present surface-treating agent is impregnated into a porous material, such as a porous ceramic material, a metal fiber, such as a flocculated steel wool to obtain a pellet. The pellet may be used, for example, in vacuum deposition.

(Article)

An article of the present disclosure (the present article) includes a base material, and a layer (surface-treating layer) on a surface of the base material and formed of the present silane compound (or a surface-treating agent containing the present silane compound).

The present surface-treating layer has good UV durability, and further can have an effect of suppressing lubricity, antifouling property (for example, preventing adhesion of fouling such as fingerprints), chemical resistance, high friction durability, water-repellency, oil-repellency, heat resistance, hydrolysis resistance and moisture-proof property.

The surface-treating layer preferably has a contact angle of water on a surface thereof of ≥ 100°.

The contact angle of water refers to a static contact angle of water on the surface of the article, and is a numerical value measured with 2 μL of water in an environment of 21°C and 65% humidity. The contact angle of water is preferably ≥ 100°, and more preferably ≥ 110°.

The contact angle of n-hexadecane on the surface of the surface-treating layer is preferably ≥ 50°, and more preferably ≥ 60°. The contact angle of n-hexadecane refers to a static contact angle of n-hexadecane on the surface of an article, and is a numerical value measured with 2 μL of water in an environment of 21°C and 65% humidity.

The ratio (i):(ii) of (i) the contact angle of water on the surface of the surface-treating layer after irradiation with ultraviolet rays of 310 nm at an illumination intensity of 0.63 $W/m^2$ for 96 hours to (ii) the contact angle of water on the surface (contact angle of water on the surface before ultraviolet irradiation), is preferably ≥ 83%, and more preferably ≥ 85%. The method of measuring the contact angle of water is as described above.

In one embodiment, the contact angle of water on the surface of the surface-treating layer is ≥ 100°, and preferably ≥ 110°, and
the ratio of the contact angle of water on the surface after irradiation with ultraviolet rays of 310 nm at an illumination intensity of 0.63 $W/m^2$ for 96 hours to the contact angle of water is ≥ 83%, and more preferably ≥ 85%.

The dynamic friction coefficient is a value measured in accordance with ASTM D4917. The dynamic friction coefficient is preferably ≥ 0.1, more preferably ≥ 0.2, and preferably ≤ 0.5, more preferably ≤ 0.4. The dynamic friction coefficient may be, for example, 0.1-0.5, or 0.15-0.35.

The contact angle of water on the surface of the present article is ≥ 100°, preferably ≥ 110°,

> the value of contact angle after 96 hours of cumulative UV irradiation time/the value of contact angle after 0 hours of UV irradiation time) is preferably ≥ 83%, and more preferably ≥ 85%, and
> the dynamic friction coefficient on the surface is particularly preferably 0.15-0.35. The contact angle of n-hexadecane on the surface of the article of the present disclosure is particularly preferably ≥ 60°.

The article may be produced, for example, as follows.

First, the base material is provided. The base material usable in the present disclosure may be composed of any suitable material such as a glass, a resin (may be a natural or synthetic resin such as a common plastic material, and may be in form of a plate, a film, or others), a metal (may be a simple substance of a metal such as aluminum, copper, or iron, or a complex such as alloy), a ceramic, a semiconductor (e.g. silicon or germanium), a fiber (e.g. a fabric or a non-woven fabric), a fur, a leather, a wood, a pottery, a stone or an architectural member.

The glass is preferably a sapphire glass, a soda-lime glass, an alkali aluminosilicate glass, a borosilicate glass, a non-alkaline glass, a crystal glass, or a quartz glass, and particularly preferably a chemically strengthened soda-lime glass, a chemically strengthened alkali aluminosilicate glass, and a chemically bound borosilicate glass.

The resin is preferably an acrylic resin or a polycarbonate resin.

For example, when an article to be produced is an optical member, a material constituting the surface of the base material may be a material for an optical member, for example, a glass or a transparent plastic. For example, when an article to be produced is an optical member, any layer (or coating) such as a hard coating layer or an antireflection layer may be formed on the surface (outermost layer) of the base material. The antireflection layer to be used may be either a single antireflection layer or a multi antireflection layer. Examples of inorganic materials usable in the antireflection layer include $SiO_2$, SiO, $ZrO_2$, $TiO_2$, TiO, $Ti_2O_3$, $Ti_2O_5$, $Al_2O_3$, $Ta_2O_5$, $CeO_2$, MgO, $Y_2O_3$, $SnO_2$, $MgF_2$, and $WO_3$. These inorganic materials may be used alone or in combination of two or more (for example, as a mixture). When a multi antireflection layer is used, it is preferable to use $SiO_2$ and/or SiO for the outermost layer. When an article to be produced is an optical glass component for a touch panel, the article may have a transparent electrode, for example, a thin layer comprising indium tin oxide (ITO) or indium zinc oxide, on a part of the surface of the base material (glass). Furthermore, the base material may have e.g. an insulating layer, an adhesive layer, a protecting layer, a decorated frame layer (I-CON), an atomizing layer, a hard coating layer, a polarizing film, a phase difference film and a liquid crystal display module, depending on its specific specification.

The shape of the base material is not limited. The region of the surface of the base material on which the surface-treating layer is to be formed may be at least a part of the surface of the base material, and may be appropriately determined depending on e.g. the application and the specific specification of the article to be produced.

The base material may be that of which at least a surface portion is made of a material originally having a hydroxyl group. Examples of the material include a glass, in addition, a metal (in particular, a base metal) having a natural oxidized layer or a thermal oxidized layer formed on the surface thereof, ceramics, and a semiconductor. Alternatively, when the hydroxyl groups are present but not sufficient as in the case of e.g. a resin, or when the hydroxyl group is originally absent, the hydroxyl group may be introduced on the surface of the base material, or the number of the hydroxyl group may be increased by subjecting the base material to any pretreatment. Examples of the pretreatment include a plasma treatment (for example, corona discharge) or an ion beam irradiation. The plasma treatment can introduce or increase hydroxyl groups on the surface of the base material, and can also be suitably used for cleaning the surface of the base material (removing e.g. foreign matters). Further, other examples of the pretreatment include a method in which a surface adsorbent having a carbon-carbon unsaturated bond group is formed on the surface of a base material in the form of a monolayer by a Langmuir-Blodgett method (LB method) or a chemical adsorption method in advance, and then the unsaturated bond is cleaved in an atmosphere containing e.g. oxygen or nitrogen.

Alternatively, the base material may be that of which at least a surface portion is made of a silicone compound having one or more other reactive group such as Si-H groups, or a material containing an alkoxysilane.

Next, a coating of the present surface treatment composition is formed on the surface of the base material, and the coating is post-treated, as necessary, thereby forming a surface-treating layer from the present surface treatment composition.

The formation of the coating of the present surface treatment composition may be performed by applying the present surface treatment composition on the surface of the base material so as to coat the surface. The method of coating is not limited. For example, wet coating methods and dry coating methods may be used.

Examples of the wet coating method include dip coating, spin coating, flow coating, spray coating, roll coating, gravure coating, and similar methods.

Examples of the dry coating method include deposition (usually, vacuum deposition), sputtering, CVD, and similar methods. Specific examples of the deposition method (usually, vacuum deposition) include resistance heating, electron beam, high-frequency heating using microwave, and ion beam. Specific examples of the CVD method include plasma-CVD, optical CVD and thermal CVD.

Additionally, coating may also be performed by an atmospheric pressure plasma method.

The coating formation is preferably performed such that the present surface treatment composition is present in the coating together with a catalyst for hydrolysis and dehydration condensation. Conveniently, when the wet coating method is used, after diluting the present surface treatment composition with a solvent and immediately before application to the surface of the base material, a catalyst may be added to the dilute solution of the present surface treatment composition. When the dry coating method is used, the present surface treatment composition to which a catalyst has been added may be directly used for deposition (usually, vacuum deposition) treatment or may be used as a substance in the form of a pellet for deposition (usually, vacuum deposition), wherein the substance in the form of a pellet is obtained by impregnating a porous body made of a metal such as iron or copper with the present surface treatment composition to which a catalyst has been added.

The catalyst to be used may be any suitable acid or base. The acid catalyst to be used may be e.g. acetic acid, formic acid or trifluoroacetic acid. The base catalyst to be used may be e.g. ammonia or an organic amine.

Next, the coating is post-treated as necessary. This post-treatment may be, but not limited to, a treatment in which water supply and dry heating are performed sequentially or simultaneously.

After the coating of the present surface treatment composition is formed on the surface of the base material as mentioned above, water is supplied to this coating (hereinafter, also referred to as precursor coating). The method for supplying water is not limited, and for example, a method such as dew condensation due to a temperature difference between the precursor coating (and the base material) and the ambient atmosphere or spraying of steam may be used.

Water may be supplied under an atmosphere, for example, at a temperature of 0-250°C, preferably ≥ 60°C, more preferably ≥ 100°C and preferably ≤ 180°C, more preferably ≤ 150°C. Hydrolysis can be promoted by supplying water in such a temperature range. The pressure at this time is not limited but can be conveniently atmospheric pressure.

Then, the precursor coating is heated on the surface of the base material under a dry atmosphere over 60°C. The dry heating method is not limited, and the precursor coating may be disposed together with the base material at a temperature in excess of 60°C, preferably in excess of 100°C, for example at a temperature of ≤ 250°C, preferably at ≤ 180°C, and in an atmosphere of an unsaturated steam pressure. The pressure at this time is not limited but can be conveniently atmospheric pressure.

Under such an atmosphere, the groups bonded to Si after hydrolysis rapidly undergo dehydration condensation between the PFPE-containing silane compounds. Furthermore, between the compound and the base material, a rapid reaction occurs between the group bonded to Si in the compound after hydrolysis and a reactive group present on the surface of the base material, and when the reactive group present on the surface of the base material is a hydroxyl group, dehydration condensation is caused. As a result, a bond is formed between the PFPE-containing silane compound and the base material.

The above water supply and dry heating may be continuously performed by using superheated steam.

The post-treatment may be performed as described above. It is noted that though the post-treatment may be performed in order to further increase friction durability, it is not essential for the production of articles. For example, the present surface treatment composition may be applied to the surface of the base material and then left as it is.

As described above, the layer derived from the coating of the present surface treatment composition is formed on the surface of the base material, and an article is produced. The layer derived from the surface treatment composition thus obtained has good UV durability, water-repellency, oil-repellency, antifouling property (for example, preventing adhesion of fouling such as fingerprints), chemical resistance, hydrolysis resistance, an effect of suppressing lubricity, high friction durability, heat resistance and moisture-proof property. Furthermore, this layer may further have surface lubricity (or lubricity, for example, wiping property for fouling such as fingerprints, excellent touch to fingers) and high friction durability, depending on the composition of the composition used, thus may be suitably used as a functional thin layer.

That is, the present surface treatment composition may also be used for forming an optical material having the cured product in the outermost layer.

Preferred examples of the optical material include optical materials related to displays, for example, displays such as a cathode ray tube (CRT; for example, TV, personal computer monitor), a liquid crystal display, a plasma display, an organic EL display, an inorganic thin-film EL dot matrix display, a rear projection display, a vacuum fluorescent display (VFD), a field emission display (FED; Field Emission Display), or a protective plate for those displays, or those obtained by applying an antireflection coating treatment to the surface thereof, and a wide variety of other optical materials.

The present article having the surface treatment composition is not limited to, but may be an optical member. Examples of the optical member include lenses for eyeglasses; front protective plates for displays such as PDPs and LCDs, antireflection plates, polarizing plates, anti-glare plates; touch panel sheets for devices such as mobile phones and personal digital assistants; disc surfaces of optical discs such as Blu-ray (registered trademark) discs, DVD discs, CD-R, and MO; and optical fibers.

In one embodiment, the present article may be, for example, a housing portion of electronic devices or office devices, for example, portable electronic devices (portable information terminals such as mobile phones, smartphones, and tablet terminals; electronic dictionaries, portable music recorders, voice recorders and players), watches, and charging stands (for example, a charging stand of the portable devices). Specifically, a layer of the PFPE-containing silane compound is formed on the exterior portion (preferably the outermost layer) of the housing of these devices.

The present article may be also a medical equipment or a medical material.

The thickness of the surface treatment composition layer is not limited. In the case of an optical member, the thickness of the layer is 1-50 nm, more preferably 1-30 nm, and particularly preferably 1-15 nm, from the viewpoint of slip suppression effect, optical performance, good UV durability, water-repellency, oil-repellency, antifouling property (for example, preventing adhesion of fouling such as fingerprints), chemical resistance, hydrolysis resistance, high friction durability, heat resistance, and moisture-proof property.

In another embodiment, the coating of the present surface-treating layer may be formed on the surface of the layer after separately forming a layer on the surface of the base material.

In another embodiment, the present article has a contact angle of water on a surface thereof of ≥ 100°, and

the ratio of the contact angle of water on the surface after irradiation with ultraviolet rays of 310 nm at an illumination of 0.63 $W/m^2$ for 96 hours to the contact angle of water is preferably ≥ 83%. The method of measuring the contact angle of water is as described above.

In the embodiment, more preferably, the present article has a contact angle of water of ≥ 110°.

In the embodiment, more preferably, the present article has a dynamic friction coefficient on the surface thereof of 0.1-0.5, still more preferably 0.15-0.35. The method of measuring the dynamic friction coefficient is as described above.

In the embodiment, the present article preferably has a contact angle of n-hexadecane on the surface thereof of ≥ 50°. The method of measuring the contact angle of n-hexadecane is as described above.

In the embodiment, the present article can have good UV durability, water-repellency, oil-repellency, antifouling property (for example, preventing adhesion of fouling such as fingerprints), chemical resistance, hydrolysis resistance, an effect of suppressing lubricity, high friction durability, heat resistance, and moisture-proof property.

Hereinbefore, the article obtained using the present surface treatment composition has been described in detail.

## Examples

The present invention will be described in more detail through the following Examples.

(Synthesis Example 1)

To a four necked flask of 200 mL equipped with a reflux condenser, a thermometer, and a stirrer, 32 g of perfluoropolyether modified carboxylic acid represented by the average compositional formula $CF_3CF_2CF_2O[CF(CF_3)CF_2O]_{22}CF(CF_3)COOH$, 16 g of 1,3-bis(trifluoromethyl)benzene, 0.13 g of N,N-dimethylformamide, and 2.17 g of thionyl chloride were charged, and the mixture was stirred at 90°C for 1 hour under a nitrogen gas flow. Subsequently, volatile content was distilled off under reduced pressure, and then charged to 16 g of 1,3-bis(trifluoromethyl)benzene, 1.35 g of triethylamine, and 1.73 g of $NH_2CH_2C(CH_2CH{=}CH_2)_3$, and the mixture was stirred under a nitrogen gas flow at room temperature for 6 hours. Subsequently, 30 g of perfluorohexane, 10 g of acetone, and 20 g of 3 mol/L hydrochloric acid were added and stirred for 30 minutes, and thereafter the perfluorohexane phase was separated using a separating funnel. Thereafter, the separated perfluorohexane phase was filtered, and then the volatile content was distilled off under reduced pressure to give 29.6 g of the perfluoropolyether group-containing allyl compound (A):

$$CF_3CF_2CF_2O[CF(CF_3)CF_2O]_{22}CF(CF_3)CONHCH_2C(CH_2CH{=}CH_2)_3$$

(Synthesis Example 2)

To a four necked flask of 200 mL equipped with a reflux condenser, a thermometer, and a stirrer, 29 g of the compound (A) synthesized in Synthesis Example 1, 35 ml of 1,3-bis(trifluoromethyl)benzene, and 6.7 g of trichlorosilane were charged, and the mixture was stirred at 5°C for 30 minutes under a nitrogen gas flow. Subsequently, 0.3 ml of a xylene solution containing 2% of a Pt complex of 1,3-divinyl-1,1,3,3-tetramethyldisiloxane was added, and then the temperature was raised to 60°C and the mixture was stirred for 6 hours. Thereafter, the volatile content was distilled off under reduced pressure. Subsequently, 30 ml of 1,3-bis(trifluoromethyl)benzene was added and stirred at 55°C for 10 minutes, and then a mixed solution of 0.73 g of methanol and 16.8 g of trimethyl orthoformate was added and stirred at this temperature for 2 hours. Thereafter, the volatile content was distilled off under reduced pressure to give 30.1 g of the following perfluoropolyether group-containing silane compound (B) :

$$CF_3CF_2CF_2O[CF(CF_3)CF_2O]_{22}CF(CF_3)CONHCH_2C[CH_2CH_2CH_2Si(OCH_3)_3\ ]_3$$

(Synthesis Example 3)

To a four necked flask of 200 mL equipped with a reflux condenser, a thermometer, and a stirrer, 32 g of perfluoropolyether modified carboxylic acid of the average compositional formula $CF_3CF_2CF_2O[CF(CF_3)CF_2O]_{28}CF(CF_3)COOH$, 16 g of 1,3-bis(trifluoromethyl)benzene, 0.10 g of N,N-dimethylformamide, and 1.69 g of thionyl chloride were charged, and the mixture was stirred at 90°C for 1 hour under a nitrogen gas flow. Subsequently, volatile content was distilled off under reduced pressure, 16 g of 1,3-bis(trifluoromethyl)benzene, 1.05 g of triethylamine, and 1.36 g of $NH_2CH_2C(CH_2CH{=}CH_2)_3$ were charged, and the mixture was stirred under a nitrogen gas flow at room temperature for 6 hours. Subsequently, 30 g of perfluorohexane, 10 g of acetone, and 20 g of 3 mol/L hydrochloric acid were added and

stirred for 30 minutes, and thereafter the perfluorohexane phase was separated using a separating funnel. Thereafter, the separated perfluorohexane phase was filtered, and then the volatile content was distilled off under reduced pressure to give 29.2 g of the following perfluoropolyether group-containing allyl compound (C):

$$CF_3CF_2CF_2O[CF(CF_3)CF_2O]_{28}CF(CF_3)CONHCH_2C(CH_2CH{=}CH_2)_3$$

(Synthesis Example 4)

To a four necked flask of 200 mL equipped with a reflux condenser, a thermometer, and a stirrer, 29 g of the compound (C) synthesized in Synthesis Example 3, 35 ml of 1,3-bis(trifluoromethyl)benzene, and 5.2 g of trichlorosilane were charged, and the mixture was stirred at 5°C for 30 minutes under a nitrogen gas flow. Subsequently, 0.23 ml of a xylene solution containing 2% of a Pt complex of 1,3-divinyl-1,1,3,3-tetramethyldisiloxane was added, and then the temperature was raised to 60°C and the mixture was stirred for 6 hours. Thereafter, the volatile content was distilled off under reduced pressure. Subsequently, 30 ml of 1,3-bis(trifluoromethyl)benzene was added and stirred at 55°C for 10 minutes, and then a mixed solution of 0.57 g of methanol and 13.1 g of trimethyl orthoformate was added and stirred at this temperature for 2 hours. Thereafter, the volatile content was distilled off under reduced pressure to give 29.6 g of the following perfluoropolyether group-containing silane compound (D) :

$$CF_3CF_2CF_2O[CF(CF_3)CF_2O]_{28}CF(CF_3)CONHCH_2C[CH_2CH_2CH_2Si(OCH_3)_3\,]_3$$

(Synthesis Example 5)

To a four necked flask of 200 mL equipped with a reflux condenser, a thermometer, and a stirrer, 32 g of perfluoropolyether modified carboxylic acid of the average compositional formula $CF_3CF_2CF_2O[CF(CF_3)CF_2O]_{11}CF(CF_3)COOH$, 16 g of 1,3-bis(trifluoromethyl)benzene, 0.23 g of N,N-dimethylformamide, and 3.81 g of thionyl chloride were charged, and the mixture was stirred at 90°C for 1 hour under a nitrogen gas flow. Subsequently, volatile content was distilled off under reduced pressure, 16 g of 1,3-bis(trifluoromethyl)benzene, 2.43 g of triethylamine, and 3.04 g of $NH_2CH_2C(CH_2CH{=}CH_2)_3$ were charged, and the mixture was stirred under a nitrogen gas flow at room temperature for 6 hours. Subsequently, 30 g of perfluorohexane, 10 g of acetone, and 20 g of 3 mol/L hydrochloric acid were added and stirred for 30 minutes, and thereafter the perfluorohexane phase was separated using a separating funnel. Thereafter, the separated perfluorohexane phase was filtered, and then the volatile content was distilled off under reduced pressure to give 29.4 g of the perfluoropolyether group-containing allyl compound (E):

$$CF_3CF_2CF_2O[CF(CF_3)CF_2O]_{11}CF(CF_3)CONHCH_2C(CH_2CH{=}CH_2)_3$$

(Synthesis Example 6)

To a four necked flask of 200 mL equipped with a reflux condenser, a thermometer, and a stirrer, 29 g of the compound (E) synthesized in Synthesis Example 5, 29.0 ml of 1,3-bis(trifluoromethyl)benzene, and 11.8 g of trichlorosilane were charged, and the mixture was stirred at 5°C for 30 minutes under a nitrogen gas flow. Subsequently, 0.8 ml of a xylene solution containing 2% of a Pt complex of 1,3-divinyl-1,1,3,3-tetramethyldisiloxane was added, and then the temperature was raised to 60°C and the mixture was stirred for 6 hours. Thereafter, the volatile content was distilled off under reduced pressure. Subsequently, 29.0 ml of 1,3-bis(trifluoromethyl)benzene was added and stirred at 55°C for 10 minutes, and then a mixed solution of 1.30 g of methanol and 29.2 g of trimethyl orthoformate was added and stirred at this temperature for 2 hours. Thereafter, the volatile content was distilled off under reduced pressure to give 30.0 g of the following perfluoropolyether group-containing silane compound (F):

$$CF_3CF_2CF_2O[CF(CF_3)CF_2O]_{11}CF(CF_3)CONHCH_2C[CH_2CH_2CH_2Si(OCH_3)_3\,]3$$

(Example 1)

The compound (B) obtained in Synthesis Example 2 was dissolved in hydrofluoroether (Novec HFE7200 manufactured by 3M Co., Ltd.) to a concentration of 20 wt% to prepare a surface-treating agent.

(Example 2)

A surface-treating agent was prepared in the same manner as in Example 1 except that the compound (D) obtained in Synthesis Example 4 was used instead of the compound (B).

(Example 3)

A surface-treating agent was prepared in the same manner as in Example 1 except that the compound (F) obtained in Synthesis Example 6 was used instead of the compound (B).

(Example 4)

A surface-treating agent was prepared in the same manner as in Example 1 except that the compound (G) was used instead of the compound (B).

· Compound (G)

$$CF_3CF_2CF_2O[CF(CF_3)CF_2O]_{22}CF(CF_3)CH_2OCH_2CH_2CH_2Si[CH_2CH_2CH_2Si\ (OCH_3)_3]_3$$

(Example 5)

A surface-treating agent was prepared in the same manner as in Example 1 except that the compound (H) was used instead of the compound (B).

· Compound (H)

$$CF_3CF_2CF_2O[CF(CF_3)CF_2O]_{22}CF(CF_3)C[OCH_2CH_2CH_2Si(OCH_3)_3][CH_2C\ H_2CH_2Si(OCH_3)_3]_2$$

(Comparative Examples 1-3)

Surface-treating agents were prepared in the same manner as in Example 1 except that the control compounds 1-3 were used instead of the compound (B).

· Control compound 1

$$CF_3CF_2CF_2O(CF_2CF_2CF_2O)_{30}CF_2CF_2CONHCH_2C[CH_2CH_2CH_2Si(OCH_3)_3]_3$$

· Control compound 2

$$CF_3O(CF_2CF_2O)_{22}(CF_2O)_{23}CF_2CONHCH_2C[CH_2CH_2CH_2Si(OCH_3)_3]_3$$

· Control compound 3

$$CF_3CF_2CF_2O[CF(CF_3)CF_2O]_{28}CF(CF_3)CONHCH_2CH_2CH_2Si(OCH_3)_3$$

<Evaluation>

The surface-treating agents prepared in Examples 1-5 and Comparative Examples 1-3 were vacuum-deposited on chemically strengthened glass ("Gorilla" glass manufactured by Corning, thickness 0.7 mm). The processing condition for the vacuum deposition was a pressure of $3.0 \times 10^{-3}$ Pa, a 5 nm silicon dioxide layer was formed on the surface of the chemically strengthened glass, and then 4 mg of a surface-treating agent (i.e., it contains 0.8 mg of any of compound

(B), compound (D), or control compounds 1 to 3) was deposited per sheet of the chemically strengthened glass (55 mm × 100 mm). Next, the chemically strengthened glass with a deposition coating was allowed to stand for 30 minutes in an atmosphere at a temperature of 150°C and then allowed to cool to room temperature to form a surface-treating layer.

(UV resistance evaluation)

With respect to the surface-treating layer formed above, the static contact angles of water before and after UV irradiation were measured. UV irradiation was performed using a UVB-313 lamp (manufactured by Q-Lab Corporation, 0.63 W/m$^2$ irradiance at 310 nm), with the black panel temperature of the base material being 63°C and the distance between the lamp and the surface-treating layer being 5 cm. The static contact angle of water was measured for 2 μL of water by using a contact angle measurement apparatus (manufactured by Kyowa Interface Science Co., Ltd.).

Firstly, as an initial evaluation, the static contact angle of water was measured after the surface-treating layer was formed and before UV irradiation (UV irradiation time of 0 hours). Thereafter, the static contact angle of water was measured for each surface-treating layer after UV irradiation for a predetermined time. The evaluation was performed up to a cumulative irradiation time of 96 hours. The results are shown in Table 1. Table 2 shows the ratio of the contact angle value after the cumulative UV irradiation time of 96 hours to the contact angle value after the UV irradiation time of 0 hours (value of contact angle after 96 hours of cumulative UV irradiation time/value of contact angle after 0 hours of UV irradiation time).

[Table 1]

| Cumulative UV irradiation time (hours) | | 0 | 24 | 48 | 72 | 96 |
|---|---|---|---|---|---|---|
| Example 1 | Contact angle (degrees) | 115 | 115 | 112 | 110 | 98 |
| Example 2 | Contact angle (degrees) | 115 | 115 | 113 | 111 | 100 |
| Example 3 | Contact angle (degrees) | 114 | 114 | 111 | 108 | 95 |
| Example 4 | Contact angle (degrees) | 114 | 114 | 111 | 107 | 93 |
| Example 5 | Contact angle (degrees) | 114 | 114 | 111 | 107 | 94 |
| Comparative Example 1 | Contact angle (degrees) | 114 | 114 | 111 | 105 | 86 |
| Comparative Example 2 | Contact angle (degrees) | 114 | 114 | 98 | 83 | 70 |
| Comparative Example 3 | Contact angle (degrees) | 114 | 114 | 111 | 107 | 92 |

[Table 2]

| Cumulative UV irradiation time (hours) | | 0 | 24 | 48 | 72 | 96 |
|---|---|---|---|---|---|---|
| Example 1 | Ratio of contact angle (%) | 100.0 | 100.0 | 97.4 | 95.7 | 85.2 |
| Example 2 | Ratio of contact angle (%) | 100.0 | 100.0 | 98.3 | 96.5 | 87.0 |
| Example 3 | Ratio of contact angle (%) | 100.0 | 100.0 | 97.4 | 94.7 | 83.3 |
| Example 4 | Ratio of contact angle (%) | 100.0 | 100.0 | 97.4 | 93.9 | 81.6 |
| Example 5 | Ratio of contact angle (%) | 100.0 | 100.0 | 97.4 | 93.9 | 82.4 |
| Comparative Example 1 | Ratio of contact angle (%) | 100.0 | 100.0 | 97.4 | 92.1 | 75.4 |
| Comparative Example 2 | Ratio of contact angle (%) | 100.0 | 100.0 | 86.0 | 72.8 | 61.4 |
| Comparative Example 3 | Ratio of contact angle (%) | 100.0 | 100.0 | 97.4 | 93.9 | 80.7 |

(Measurement of static contact angle of n-hexadecane)

With respect to the surface-treating layers formed above, the static contact angle of n-hexadecane was measured. The static contact angle of n-hexadecane was measured for 2 μL of n-hexadecane by using a contact angle measurement apparatus (manufactured by Kyowa Interface Science Co., Ltd.). The results are shown in the table below.

[Table 3]

| | Contact angle of n-hexadecane (degrees) |
|---|---|
| Example 1 | 70 |
| Example 2 | 70 |
| Example 3 | 70 |
| Example 4 | 68 |
| Example 5 | 66 |

· Surface lubricity evaluation (measurement of dynamic friction coefficient)

The dynamic friction coefficient (-) of the base material having the surface-treating layer on the surface formed above was measured in accordance with ASTM D4917 using paper as a friction block using a surface measuring instrument (manufactured by Labthink Instruments Co. Ltd., FPT-1) .

Specifically, the base material having the surface-treating layer was disposed horizontally, paper (2 cm × 2 cm) serving as a friction block was brought into contact with the exposed upper surface of the surface-treating layer, and a load of 200 gf was applied thereon. Thereafter, the paper serving as a friction block was moved in parallel at a speed of 200 mm/sec under the load, and the dynamic friction coefficient was measured. The results are shown in the table below.

[Table 4]

| | Dynamic friction coefficient (-) |
|---|---|
| Example 1 | 0.26 |
| Example 2 | 0.23 |
| Example 3 | 0.28 |
| Example 4 | 0.24 |
| Example 5 | 0.23 |
| Comparative Example 1 | 0.07 |
| Comparative Example 2 | 0.03 |
| Comparative Example 3 | 0.22 |

· Evaluation of Friction Durability

The surface-treating layers formed using the surface-treating agents of Examples 1-5 and Comparative Example 3 described above were evaluated for friction durability by an eraser friction durability test.

First, as an initial evaluation, the static contact angle of water in the eraser friction durability test was measured (number of frictions: 0).

Thereafter, the base material on which the surface-treating layer was formed was horizontally arranged, and then, an eraser (manufactured by minoan, model number: MB006004, diameter 0.6 cm) was brought into contact with the surface of the surface-treating layer, and a load of 1000 gf was applied thereon. Then, the eraser was reciprocated at a rate of 20 mm/sec while applying the load. The static contact angle (degree) of water was measured every 1000 reciprocations. The results are shown in the table below. In the table, "-" indicates that the measurement was not performed.

[Table 5]

| Number of frictions (times) | | 0 | 1000 | 2000 | 3000 |
|---|---|---|---|---|---|
| Example 1 | Contact angle (degrees) | 115 | 102 | 84 | 58 |
| Example 2 | Contact angle (degrees) | 115 | 106 | 95 | 79 |
| Example 3 | Contact angle (degrees) | 114 | 95 | 78 | 43 |

(continued)

| Number of frictions (times) | | 0 | 1000 | 2000 | 3000 |
|---|---|---|---|---|---|
| Example 4 | Contact angle (degrees) | 114 | 96 | 80 | 45 |
| Example 5 | Contact angle (degrees) | 114 | 93 | 77 | 40 |
| Comparative Example 3 | Contact angle (degrees) | 114 | 88 | 43 | - |

Industrial Applicability

The present disclosure can be suitably used for forming a surface-treating layer on the surface of various base materials, particularly articles (for example, electronic devices) for which e.g. UV durability and slip suppression effect are required.

## Claims

1. A compound which is a fluoro(poly)ether group-containing silane compound of any of the formulae (A1), (A2), (B1), (B2), (C1) or (C2):

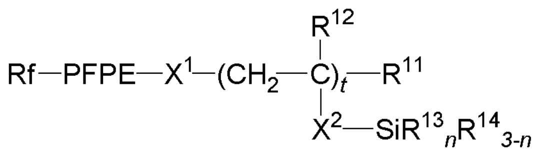

(A1)

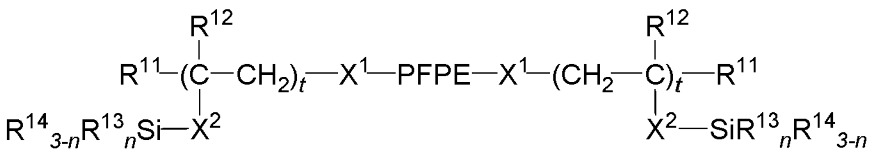

(A2)

wherein, each independently at each occurrence:

PFPE is $-(OC_3F_6)_d-$ wherein the repeating unit- $(OC_3F_6)$ - has a branched structure, and d is an integer of 2-200;
Rf is $C_{1-16}$-alkyl optionally substituted by F;
$X^1$ is $-(R^{31})_{p'}-((X^b)_{l'})_{q'}-$; wherein, each independently at each occurrence,

$R^{31}$ is a single bond, $-(CH_2)_{s'}-$ optionally substituted by one or more F, or o-, m-, or p-phenylene;
s' is an integer of 1-20;
$X^b$ is -O-, $-(OR^{35})_{n4}-$, -S-, o-, m- or p-phenylene, -C(O)O-, $-Si(R^{33})_2-$, $-(Si(R^{33})_2O)_{m'}-Si(R^{33})_2-$, $-CON(R^{34})-$, $-O-CON(R^{34})-$, $-N(R^{34})-$ or $-(CH_2)_{n'}-$,
wherein

$R^{33}$ is phenyl, a $C_{1-6}$-alkyl or $C_{1-6}$-alkoxy;
$R^{34}$ is H, phenyl or $C_{1-6}$-alkyl;
$R^{35}$ is $C_{1-6}$-alkylene;
n4 is an integer of 1-5;
m' is an integer of 1-100;
n' is an integer of 1-20;

l' is an integer of 1-10;
p' is 0 or 1;
q' is 0 or 1;

at least one of p' and q' is 1, and the order of therepeating units in parentheses is arbitrary;
$R^{11}$ is H or halogen;
$R^{12}$ is H or $C_{1-20}$-alkyl;
$X^2$ is a single bond or a divalent organic group;
$R^{13}$ is OH or a hydrolyzable group;
$R^{14}$ is H or $C_{1-22}$-alkyl;
n is an integer of 1-3 for each $(-SiR^{13}_{n}R^{14}_{3-n})$ unit;
t is an integer of 2-10;
in the formulae (A1) and (A2), there are at least two $SiR^{13}$;

$(Rf\text{-}PFPE)_{\beta'}\text{-}X^3\text{-}(SiR^a_3)_\beta$ (B1)

$(R^a_3Si)_\beta\text{-}X^3\text{-}PFPE\text{-}X^3\text{-}(SiR^a_3)_\beta$ (B2)

wherein PFPE and Rf are as defined above and, each independently at each occurrence:

$X^3$ is a single bond or a 2- to 10-valent organic group;
β is an integer of 1-9;
β' is an integer of 1-9;
$R^a$ is $-Z^3SiR^{72}_{q1}R^{73}_{r1}$; wherein, each independently at each occurrence,

$Z^3$ is a divalent organic group and does not include a group which forms a siloxane bond together with a Si atom;
$R^{72}$ is OH or a hydrolyzable group;
$R^{73}$ is H or $C_{1-20}$-alkyl;
*q*1 is 2 or 3;
r1 is 0 or 1;

(q1+r1) = 3 in each $(-Z^3\text{-}SiR^{72}_{q1}R^{73}_{r1})$;

$Rf\text{-}PFPE\text{-}X^5\text{-}CR^e_3$ (C1)

$R^e_3C\text{-}X^5\text{-}PFPE\text{-}X^5\text{-}CR^e_3$ (C2)

wherein PFPE and Rf are as defined above, $X^5$ is as defined for $X^1$ in formulae (A1) and (A2) above and, each independently at each occurrence:

$R^e$ is $-Y\text{-}SiR^{85}_{n2}R^{86}_{3-n2}$;

Y is a divalent organic group;
$R^{85}$ is OH or a hydrolyzable group;
$R^{86}$ is H or $C_{1-20}$-alkyl; and
n2 is an integer of 1-3 for each $(-Y\text{-}SiR^{85}_{n2}R^{86}_{3-n2})$ unit,

with the proviso that a compound of the formula

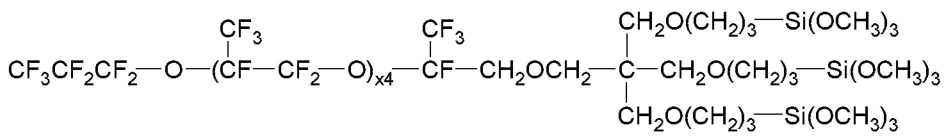

wherein x4 = 6 and is a mean value of the number of units, is excluded

2. The compound of claim 1, wherein PFPE is $-(OCF(CF_3)CF_2)_d-$ wherein d is an integer of 2-200.

3. The compound of claim 1 or 2, wherein β and β' are 1.

4. The compound of any of claims 1-3, wherein $X^1$, $X^3$, and $X^5$ each independently are $C_{1-6}$-alkylene, $-X^{10}-CON(R^{34})-X^{11}-$ or $-X^{10}-(OR^{35})_{n4}-X^{11}-$, wherein, each independently at each occurrence:

   $X^{10}$ is a single bond or a divalent organic group;
   $X^{11}$ is a single bond, O or a divalent organic group;
   $R^{34}$ is H, phenyl or $C_{1-6}$-alkyl;
   $R^{35}$ is $C_{1-6}$-alkylene; and
   n4 is an integer of 1-5.

5. The compound of claim any of claims 1-4, wherein *q*1 is 2 or 3.

6. The compound of any of claims 1-5, which is a compound of formula (B1), (B2), (C1) or (C2).

7. The compound of any of claims 1-6, which is a compound of formula (B1).

8. The compound of any of claims 1-6, which is a compound of formula (C1) or (C2), and preferably of formula (C1).

9. A surface-treating agent comprising the compound of any of claims 1-8.

10. The surface-treating agent of claim 9, further comprising a solvent.

11. The surface-treating agent of claim 9 or 10, further comprising one or more other components selected from a fluorine-containing oil, a silicone oil, an alcohol, a catalyst, a transition metal, a halide ion, and a compound containing an atom with an unshared electron pair in a molecular structure.

12. The surface-treating agent of any of claims 9-11, which is used as an antifouling coating agent or a waterproof coating agent.

13. A pellet comprising the surface-treating agent of any of claims 9-12.

14. An article comprising a base material, and, on a surface of the base material, a layer formed of the surface-treating agent of any of claims 9-12.

15. The article of claim 14, which has a contact angle of water on a surface of ≥ 100°, and the ratio of the contact angle of water after irradiation with UV-light of 310 nm at an illumination intensity of 0.63 $W/m^2$ for 96 hours to the contact angle prior to irradiation is ≥ 83%.

16. A compound of formula (c1-3) or (c2-3):

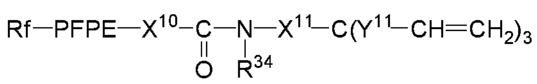

(c1-3)

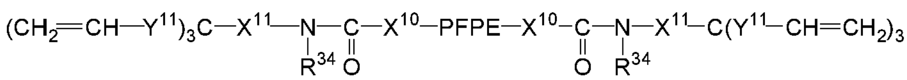

(c2-3)

wherein, each independently at each occurrence,

   Rf is $C_{1-16}$-alkyl optionally substituted by F;
   PFPE is $(-OCF(CF_3)CF_2-)_d$ wherein d is an integer of 2-200;
   $X^{10}$ is a single bond or a divalent organic group;
   $R^{34}$ is H, phenyl or $C_{1-6}$-alkyl;
   $X^{11}$ is a single bond or a divalent organic group; and
   $Y^{11}$ is a single bond or a divalent organic group.

## Patentansprüche

1. Verbindung, die eine Fluor(poly)ethergruppen-haltige Silanverbindung einer der Formeln (A1), (A2), (B1), (B2), (C1) oder (C2) ist:

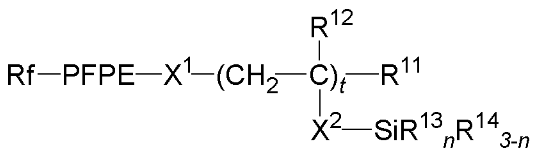

(A1)

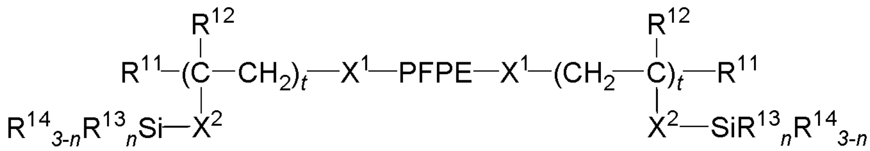

(A2)

worin, jeweils unabhängig bei jedem Auftreten:

PFPE $-(OC_3F_6)_d-$ ist, worin die Wiederholungseinheit-$(OC_3F_6)$- eine verzweigte Struktur aufweist und d eine ganze Zahl von 2-200 ist;
Rf $C_{1-16}$-Alkyl ist, das gegebenenfalls durch F substituiert ist;
$X^1$ $-(R^{31})_{p'}-((X^b)_{l'})_{q'}-$ ist; worin, jeweils unabhängig bei jedem Auftreten,

$R^{31}$ eine Einfachbindung, $-(CH_2)_{s'}-$, das gegebenenfalls durch ein oder mehrere F substituiert ist, oder o-, m-, oder p-Phenylen ist;
s' eine ganze Zahl von 1-20 ist;
$X^b$ -O-, $-(OR^{35})_{n4}-$, -S-, o-, m- oder p-Phenylen, -C(O)O-, $-Si(R^{33})_2-$, $-(Si(R^{33})_2O)_{m'}-Si(R^{33})_2-$, $-CON(R^{34})-$, $-O-CON(R^{34})-$, $-N(R^{34})-$ oder $-(CH_2)_{n'}-$ ist, worin

$R^{33}$ Phenyl, ein $C_{1-6}$-Alkyl oder $C_{1-6}$-Alkoxy ist;
$R^{34}$ H, Phenyl oder $C_{1-6}$-Alkyl ist;
$R^{35}$ $C_{1-6}$-Alkylen ist;
n4 eine ganze Zahl von 1-5 ist;
m' eine ganze Zahl von 1-100 ist;
n' eine ganze Zahl von 1-20 ist;

l' eine ganze Zahl von 1-10 ist;
p' 0 oder 1 ist;
q' 0 oder 1 ist;

mindestens eines von p' und q' 1 ist, und die Reihenfolge der Wiederholungseinheiten in Klammern beliebig ist;
$R^{11}$ H oder Halogen ist;
$R^{12}$ H oder $C_{1-20}$-Alkyl ist;
$X^2$ eine Einfachbindung oder eine zweiwertige organische Gruppe ist;
$R^{13}$ OH oder eine hydrolysierbare Gruppe ist;
$R^{14}$ H oder $C_{1-22}$-Alkyl ist;
n eine ganze Zahl von 1-3 für jede $(-SiR^{13}{}_nR^{14}{}_{3-n}$-Einheit ist;
t eine ganze Zahl von 2-10 ist;

in den Formeln (A1) und (A2), mindestens zwei $SiR^{13}$ vorhanden sind;

$$(Rf\text{-}PFPE)_{\beta'}\text{-}X^3\text{-}(SiR^a{}_3)_\beta \qquad (B1)$$

$$(R^a{}_3Si)_\beta\text{-}X^3\text{-}PFPE\text{-}X^3\text{-}(SiR^a{}_3)_\beta \qquad (B2)$$

worin PFPE und Rf wie oben definiert sind und, jeweils unabhängig bei jedem Auftreten:

$X^3$ eine Einfachbindung oder eine 2- bis 10-wertige organische Gruppe ist;
β eine ganze Zahl von 1-9 ist;
β' eine ganze Zahl von 1-9 ist;
$R^a$ $-Z^3-SiR^{72}_{q1}R^{73}_{r1}$ ist; worin, jeweils unabhängig bei jedem Auftreten,

$Z^3$ eine zweiwertige organische Gruppe ist und keine Gruppe enthält, die zusammen mit einem Si-Atom eine Siloxanbindung bildet;
$R^{72}$ OH oder eine hydrolysierbare Gruppe ist;
$R^{73}$ H oder $C_{1-20}$-Alkyl ist;
q1 2 oder 3 ist;
r1 0 oder 1 ist;

$(q1+r1) = 3$ in jedem $(-Z^3-SiR^{72}_{q1}R^{73}_{r1})$;

$$Rf-PFPE-X^5-CR^e_3 \quad (C1)$$

$$R^e_3C-X^5-PFPE-X^5-CR^e_3 \quad (C2)$$

worin PFPE und Rf wie oben definiert sind, $X^5$ wie für $X^1$ in den Formel (A1) und (A2) oben definiert ist und, jeweils unabhängig bei jedem Auftreten:

$R^e$ $-Y-SiR^{85}_{n2}R^{86}_{3-n2}$ ist;

Y eine zweiwertige organische Gruppe ist;
$R^{85}$ OH oder eine hydrolysierbare Gruppe ist;
$R^{86}$ H oder $C_{1-20}$-Alkyl ist; und
n2 eine ganze Zahl von 1-3 für jede $(-Y-SiR^{85}_{n2}R^{86}_{3-n2})$-Einheit ist,

mit der Maßgabe, dass eine Verbindung der Formel

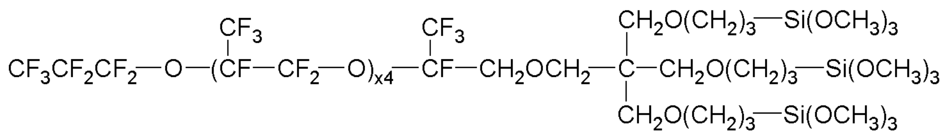

worin x4 = 6 und ein durchschnittlicher Wert der Anzahl an Einheiten ist, ausgeschlossen ist.

**2.** Verbindung gemäß Anspruch 1, worin PFPE $-(OCF(CF_3)CF_2)_d-$ ist, worin d eine ganze Zahl von 2-200 ist.

**3.** Verbindung gemäß Anspruch 1 oder 2, worin β und β' 1 sind.

**4.** Verbindung gemäß einem der Ansprüche 1-3, worin $X^1$, $X^3$ und $X^5$ jeweils unabhängig $C_{1-6}$-Alkylen, $-X^{10}-CON(R^{34})-X^{11}-$ oder $-X^{10}-(OR^{35})_{n4}-X^{11}-$ sind, worin, jeweils unabhängig bei jedem Auftreten:

$X^{10}$ eine Einfachbindung oder eine zweiwertige organische Gruppe ist;
$X^{11}$ eine Einfachbindung, O oder eine zweiwertige organische Gruppe ist;
$R^{34}$ H, Phenyl oder $C_{1-6}$-Alkyl ist;
$R^{35}$ $C_{1-6}$-Alkylen ist; und
n4 eine ganze Zahl von 1-5 ist.

**5.** Verbindung gemäß einem der Ansprüche 1-4, worin *q*1 2 oder 3 ist.

**6.** Verbindung gemäß einem der Ansprüche 1-5, die eine Verbindung der Formel (B1), (B2), (C1) oder (C2) ist.

7. Verbindung gemäß einem der Ansprüche 1-6, die eine Verbindung der Formel (B1) ist.

8. Verbindung gemäß einem der Ansprüche 1-6, die eine Verbindung der Formel (C1) oder (C2) und vorzugsweise der Formel (C1) ist.

9. Oberflächenbehandlungsmittel, umfassend die Verbindung gemäß einem der Ansprüche 1-8.

10. Oberflächenbehandlungsmittel gemäß Anspruch 9, ferner umfassend ein Lösungsmittel.

11. Oberflächenbehandlungsmittel gemäß Anspruch 9 oder 10, ferner umfassend eine oder mehrere andere Komponenten, ausgewählt aus einem Fluor-haltigen Öl, einem Silikonöl, einem Alkohol, einem Katalysator, einem Übergangsmetall, einem Halogenidion und einer Verbindung, die ein Atom mit einem ungeteilten Elektronenpaar in einer Molekülstruktur enthält.

12. Oberflächenbehandlungsmittel gemäß einem der Ansprüche 9-11, die als Antifouling-Beschichtungsmittel oder als wasserfestes Beschichtungsmittel verwendet wird.

13. Pellet, umfassend das Oberflächenbehandlungsmittel gemäß einem der Ansprüche 9-12.

14. Gegenstand, umfassend ein Basismaterial und, auf einer Oberfläche des Basismaterials, eine aus dem Oberflächenbehandlungsmittel gemäß einem der Ansprüche 9-12 geformte Schicht.

15. Gegenstand gemäß Anspruch 14, der auf einer Oberfläche einen Wasserkontaktwinkel von ≥ 100° aufweist und das Verhältnis des Wasserkontaktwinkels nach Bestrahlung mit UV-Licht von 310 nm bei einer Beleuchtungsintensität von 0,63 $W/m^2$ für 96 Stunden zum Kontaktwinkel vor der Bestrahlung ≥ 83% beträgt.

16. Verbindung der Formel (c1-3) oder (c2-3):

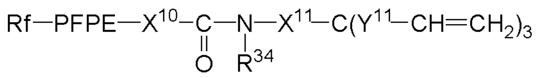

(c1-3)

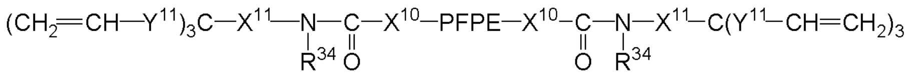

(c2-3)

worin, jeweils unabhängig bei jedem Auftreten,

Rf $C_{1-16}$-Alkyl ist, das gegebenenfalls durch F substituiert ist;
PFPE $(-OCF(CF_3)CF_2-)_d$ ist, worin d eine ganze Zahl von 2-200 ist;
$X^{10}$ eine Einfachbindung oder eine zweiwertige organische Gruppe ist;
$R^{34}$ H, Phenyl oder $C_{1-6}$-Alkyl ist;
$X^{11}$ eine Einfachbindung oder eine zweiwertige organische Gruppe ist; und
$Y^{11}$ eine Einfachbindung oder eine zweiwertige organische Gruppe ist.

**Revendications**

1. Composé qui est un composé silane contenant un groupe fluoro(poly)éther selon l'une quelconque des formules (A1), (A2), (B1), (B2), (C1) ou (C2) :

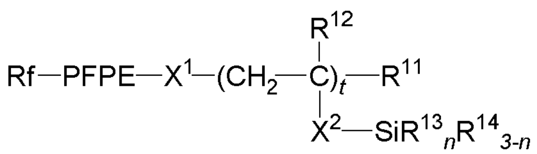

(A1)

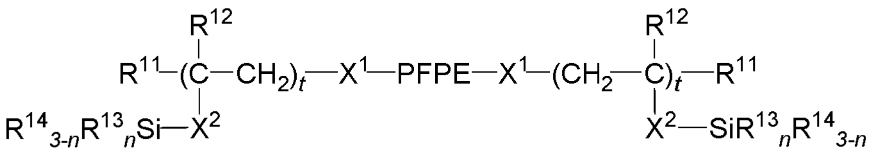

(A2)

dans lequel, chacun indépendamment à chaque occurrence :

PFPE est $-(OC_3F_6)_d-$ dans lequel l'unité de répétition $-(OC_3F_6)-$ présente une structure ramifiée, et d est un nombre entier de 2 à 200 ;
Rf est un alkyle en $C_{1-16}$ facultativement substitué par F ;
$X^1$ est $-(R^{31})_{p'}-((X^b)_{l'})_{q'}-$ ; dans lequel, chacun indépendamment à chaque occurrence,

$R^{31}$ est une liaison simple, $-(CH_2)_{s'}-$ facultativement substitué par un ou plusieurs F, ou un o-, m- ou p-phénylène ;
s' est un nombre entier de 1 à 20 ;
$X^b$ est -O-, $-(OR^{35})_{n4}-$, -S-, un o-, m- ou p-phénylène, -C(O)O-, $-Si(R^{33})_2-$, $-(Si(R^{33})_2O)_{m'}-Si(R^{33})_2-$, $-CON(R^{34})-$, $-O-CON(R^{34})-$, $-N(R^{34})-$ ou $-(CH_2)_{n'}-$, dans lequel

$R^{33}$ est un phényle, un alkyle en $C_{1-6}$, ou un alcoxy en $C_{1-6}$ ;
$R^{34}$ est H, un phényle ou un alkyle en $C_{1-6}$ ;
$R^{35}$ est un alkylène en $C_{1-6}$ ;
n4 est un nombre entier de 1 à 5 ;
m' est un nombre entier de 1 à 100 ;
n' est un nombre entier de 1 à 20 ;
l' est un nombre entier de 1 à 10 ;
p' est 0 ou 1 ;
q' est 0 ou 1 ;

au moins l'un parmi p' et q' est 1, et l'ordre des unités de répétition entre parenthèses est arbitraire ;
$R^{11}$ est H ou un halogène ;
$R^{12}$ est H ou un alkyle en $C_{1-20}$- ;
$X^2$ est une liaison simple ou un groupe organique divalent ;
$R^{13}$ est OH ou un groupe hydrolysable ;
$R^{14}$ est H ou un alkyle en $C_{1-22}$- ;
*n* est un nombre entier de 1 à 3 pour chaque unité $(-SiR^{13}_nR^{14}_{3-n})$ ;
*t* est un nombre entier de 2 à 10 ; dans les formules (A1) et (A2), il y a au moins deux $SiR^{13}$ ;

$$(Rf\text{-}PFPE)_{\beta'}\text{-}X^3\text{-}(SiR^a_3)_\beta \quad (B1)$$

$$(R^a_3Si)_\beta\text{-}X^3\text{-}PFPE\text{-}X^3\text{-}(SiR^a_3)_\beta \quad (B2)$$

dans lequel PFPE et Rf sont tels que précédemment définis et, chacun indépendamment à chaque occurrence :
$X^3$ est une liaison simple ou un groupe organique divalent à décavalent ;
$\beta$ est un nombre entier de 1 à 9 ;
$\beta'$ est un nombre entier de 1 à 9 ;
$R^a$ est $-Z^3-SiR^{72}_{q1}R^{73}_{r1}$ ; dans lequel, chacun indépendamment à chaque occurrence,

$Z^3$ est un groupe organique divalent et n'inclut pas un groupe formant une liaison siloxane conjointement avec un atome de Si ;
$R^{72}$ est OH ou un groupe hydrolysable ;
$R^{73}$ est H ou un alkyle en $C_{1-20}$- ;
*q*1 est 2 ou 3 ;
*r*1 est 0 ou 1 ;

$$(q1 + r1) = 3$$

dans chaque ($-Z^3-SiR^{72}_{q1}R^{73}_{r1}$);

$$Rf\text{-}PFPE\text{-}X^5\text{-}CR^e_3 \qquad (C1)$$

$$R^e_3C\text{-}X^5\text{-}PFPE\text{-}X^5\text{-}CR^e_3 \qquad (C2)$$

dans lequel PFPE et Rf sont tels que précédemment définis, $X^5$ est tel que défini pour $X^1$ dans les formules (A1) et (A2) ci-dessus et, chacun indépendamment à chaque occurrence :
$R^e$ est $-Y-SiR^{85}_{n2}R^{86}_{3-n2}$ ;

Y est un groupe organique divalent ;
$R^{85}$ est OH ou un groupe hydrolysable ;
$R^{86}$ est H ou un alkyle en $C_{1-20}$ ; et
*n*2 est un nombre entier de 1 à 3 pour chaque unité ($-Y-SiR^{85}_{n2}R^{86}_{3-n2}$),

à condition qu'un composé de formule

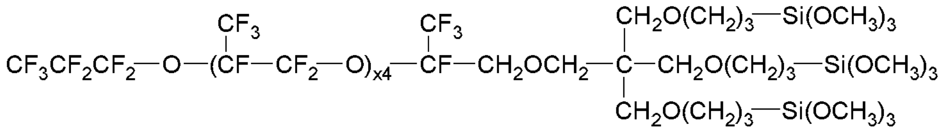

dans lequel x4 = 6 est une valeur moyenne du nombre d'unités, soit exclu

**2.** Composé selon la revendication 1, dans lequel PFPE est $-(OCF(CF_3)CF_2)_d-$ dans lequel *d* est un nombre entier de 2 à 200.

**3.** Composé selon la revendication 1 ou la revendication 2, dans lequel β et β' sont 1.

**4.** Composé selon l'une quelconque des revendications 1 à 3, dans lequel $X^1$, $X^3$ et $X^5$ sont chacun indépendamment un alkylène en $C_{1-6}$, $-X^{10}-CON(R^{34})-X^{11}-$ ou $-X^{10}-(OR^{35})_{n4}-X^{11}-$, dans lequel, chacun indépendamment à chaque occurrence :

$X^{10}$ est une liaison simple ou un groupe organique divalent ;
$X^{11}$ est une liaison simple, O ou un groupe organique divalent ;
$R^{34}$ est H, un phényle ou un alkyle en $C_{1-6}$ ;
$R^{35}$ est un alkylène en $C_{1-6}$ ; et
*n*4 est un nombre entier de 1 à 5.

**5.** Composé selon l'une quelconque des revendications 1 à 4, dans lequel *q*1 est 2 ou 3.

**6.** Composé selon l'une quelconque des revendications 1 à 5, qui est un composé de formule (B1), (B2), (C1) ou (C2).

**7.** Composé selon l'une quelconque des revendications 1 à 6, qui est un composé de formule (B1).

**8.** Composé selon l'une quelconque des revendications 1 à 6, qui est un composé de formule (C1) ou (C2), et de préférence de formule (C1).

**9.** Agent de traitement de surface comprenant le composé selon l'une quelconque des revendications 1 à 8.

**10.** Agent de traitement de surface selon la revendication 9, comprenant en outre un solvant.

**11.** Agent de traitement de surface selon la revendication 9 ou la revendication 10, comprenant en outre un ou plusieurs autres composants sélectionnés parmi une huile contenant du fluor, une huile silicone, un alcool, un catalyseur, un métal de transition, un ion halogénure et un composé contenant un atome ayant une paire d'électrons non partagée dans une structure moléculaire.

**12.** Agent de traitement de surface selon l'une quelconque des revendications 9 à 11, qui est utilisé en tant qu'agent de revêtement antisalissure ou agent de revêtement étanche à l'eau.

**13.** Pastille comprenant l'agent de traitement de surface selon l'une quelconque des revendications 9 à 12.

**14.** Article comprenant un matériau de base, et, sur une surface du matériau de base, une couche formée à partir de l'agent de traitement de surface selon l'une quelconque des revendications 9 à 12.

**15.** Article selon la revendication 14, qui présente un angle de contact de l'eau sur une surface de >_ 100°, et le rapport de l'angle de contact de l'eau après irradiation avec une lumière UV de 310 nm à une intensité d'éclairage de 0,63 W/$m^2$ pendant 96 heures à l'angle de contact avant irradiation est ≥ 83 %.

**16.** Composé de formule (c1-3) ou (c2-3) :

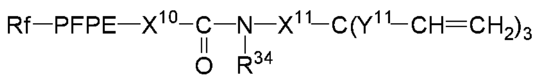

(c1-3)

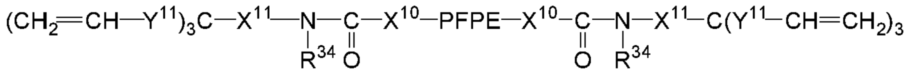

(c2-3)

dans lequel, chacun indépendamment à chaque occurrence,

Rf est un alkyle en $C_{1-16}$ facultativement substitué par F ;
PFPE est $(-OCF(CF_3)CF_2-)_d$ dans lequel d est un nombre entier de 2 à 200 ;
$X^{10}$ est une liaison simple ou un groupe organique divalent ;
$R^{34}$ est H, un phényle ou un alkyle en $C_{1-6}$ ;
$X^{11}$ est une liaison simple ou un groupe organique divalent ; et
$Y^{11}$ est une liaison simple ou un groupe organique divalent.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015221888 A
- WO 2015200003 A
- WO 2017038305 A
- EP 1923434 A
- JP 2002348370 A
- EP 3085749 A
- EP 3747931 A
- EP 3345912 A
- WO 2014069592 A


### Non-patent literature cited in the description


- **Y. ITAMI et al.** *Surface Coatings Int. Part B : Coatings,* 2006, vol. 89, 255-258